# EUROPEAN PATENT APPLICATION

(11) **EP 1 624 062 A2**
(43) Date of publication of application: **08.02.2006**
(21) Application number: 05020876.8
(22) Date of filing: 26.10.2001
(51) Int. Cl.: C12N 15/12, C07K 14/705, C07K 14/72

(54) **Human 7tm proteins and polynucleotides encoding them**

(30) Priority: 27.10.2000 US 243948 P; 30.10.2000 US 244291 P
(62) Divisional of application: 01272483.7
(71) Applicant: Lexicon Genetics Incorporated, The Woodlands, TX 77381-1160 (US)
(72) Inventor: Walke, D. Wade, Spring, TX 77389 (US); Scoville, John, Houston, TX 77024 (US)
(74) Representative: Silveston, Judith

(57) **Abstract**

The nucleotide and amino acid sequences of two novel human G protein coupled receptors are described.

## Description

The present application claims the benefit of U.S. Provisional Application Numbers 60/243,948, which was filed on October 27, 2000, and 60/244,291, which was filed on October 30, 2000, both of which are herein incorporated by reference in their entirety.

### 1. INTRODUCTION

The present invention relates to the discovery, identification and characterization of novel human polynucleotides that encode membrane associated proteins and receptors. The invention encompasses the described polynucleotides, host cell expression systems, the encoded proteins, fusion proteins, polypeptides and peptides, antibodies to the encoded proteins and peptides, and genetically engineered animals that lack the disclosed genes, or overexpress the disclosed genes, or antagonists and agonists of the proteins, and other compounds that modulate the expression or activity of the proteins encoded by the disclosed genes that can be used for diagnosis, drug screening, clinical trial monitoring, the treatment of physiological or behavioral disorders, and/or cosmetic or nutriceutical applications.

### 2. BACKGROUND OF THE INVENTION

Membrane receptor proteins can serve as integral components of cellular mechanisms for sensing their environment, and maintaining cellular homeostasis and function. Accordingly, membrane receptor proteins are often involved in transduction pathways that control cell physiology, chemical communication, and gene expression. A particularly relevant class of membrane receptors are those typically characterized by the presence of 7 conserved transmembrane domains that are interconnected by nonconserved hydrophilic loops. Such, "7TM receptors" include a superfamily of receptors known as G-protein coupled receptors (GPCRs). GPCRs are typically involved in transduction pathways involving G-proteins or PPG proteins. As such, the GPCR family includes many receptors that are known to serve as drug targets for therapeutic agents.

### 3. SUMMARY OF THE INVENTION

The present invention relates to the discovery, identification, and characterization of nucleotides that encode novel GPCRs, and the corresponding novel GPCR (NGPCR) amino acid sequences. The NGPCRs described for the first time herein are transmembrane proteins that span the cellular membrane and are involved in signal transduction after ligand binding. The described NGPCRs have structural motifs found in the 7TM receptor family. Expression of the described NGPCRs can be detected in a variety of human cells.

The novel human GPCR sequences described herein encode proteins of 640 and 578 amino acids in length (see respectively SEQ ID NOS:2 and 4). The described NGPCRs have multiple transmembrane regions (of about 20-30 amino acids) characteristic of 7TM proteins, as well as several predicted cytoplasmic domains.

Additionally contemplated are "knockout" ES cells that have been engineered using conventional methods (see, for example, PCT Patent Application No. PCT/US98/03243, filed February 20, 1998, herein incorporated by reference). Accordingly, an additional aspect of the present invention includes knockout cells and animals having genetically engineered mutations in the sequences encoding the presently described NGPCRs.

The invention encompasses the nucleotides presented in the Sequence Listing, expression vectors that have been engineered to incorporate one or more of the nucleotides presented in the Sequence Listing, host cells expressing such nucleotides, and the expression products of such nucleotides, and: (a) nucleotides that encode mammalian homologs of the described NGPCRs, including the specifically described human NGPCRs, and the human NGPCR gene products; (b) nucleotides that encode one or more portions of the NGPCRs that correspond to functional domains, and the polypeptide products specified by such nucleotide sequences, including, but not limited to, the novel regions of the described extracellular domain(s) (ECD), one or more transmembrane domain(s) (TM) first disclosed herein, and the cytoplasmic domain(s) (CD); (c) isolated nucleotides that encode mutants, engineered or naturally occurring, of the described NGPCRs, in which all or a part of at least one of the domains is deleted or altered, and the polypeptide products specified by such nucleotide sequences, including, but not limited to, soluble receptors in which all or a portion of a TM is deleted (in the case of the described 7TMs a soluble product can be generated by engineering a protein to include only the region upstream from the first TM such that all downstream TMs are deleted), and nonfunctional receptors in which all or a portion of one or more of the CD(s) is deleted; (d) nucleotides that encode fusion proteins containing all or a portion of the coding region from a NGPCR, or one of its domains (e.g., an extracellular domain) fused to another peptide or polypeptide; and (e) therapeutic or diagnostic derivatives of the described polynucleotides, such as oligonucleotides, antisense polynucleotides, ribozymes, dsRNA, or gene therapy constructs, comprising one or more of the sequences first disclosed in the Sequence Listing.

The invention also encompasses agonists and antagonists of the NGPCRs, including small molecules, large molecules, mutant NGPCR proteins, or portions thereof that compete with the native NGPCR, and antibodies, as well as nucleotide sequences that can be used to inhibit the expression of the described NGPCRs (e.g., antisense and ribozyme molecules, and gene or regulatory sequence replacement constructs) or to enhance the expression of the described NGPCR gene (*e.g*., expression constructs that place the described sequence under the control of a strong promoter system), and transgenic animals that express a NGPCR transgene or "knock-outs" that do not express a functional NGPCR. Knock-out mice can be produced in several ways, one of which involves the use of mouse embryonic stem cell ("ES cell") lines that contain gene trap mutations in a murine homolog of at least one of the described NGPCRs. When the unique NGPCR sequences described in SEQ ID NOS:1-5 are "knocked-out" they provide a method of identifying phenotypic expression of the particular gene, as well as a method of assigning function to previously unknown genes. In addition, animals in which the unique NGPCR sequences described in SEQ ID NOS:1-5 are "knocked-out" provide a unique source in which to elicit antibodies to homologous and orthologous proteins, which would have been previously viewed by the immune system as "self" and therefore would have failed to elicit significant antibody responses. To these ends, gene trapped knockout ES cells have been generated in murine homologs of the described NGPCRs.

Additionally, the unique NGPCR sequences described in SEQ ID NOS:1-5 are useful for the identification of protein coding sequences and mapping unique genes to one or more particular chromosome (the described NGPCRs are apparently encoded on human chromosome 6, see GENBANK accession no. AL356421). These sequences identify actual, biologically relevant, exon splice junctions, as opposed to those that might have been predicted bioinformatically from genomic sequence alone. The sequences of the present invention are also useful as additional DNA markers for restriction fragment length polymorphism (RFLP) analysis, and in forensic biology.

Further, the present invention also relates to methods of using the described NGPCR nucleotide sequences and/or NGPCR gene products for the identification of compounds that modulate, *i.e.*, act as agonists or antagonists of, NGPCR gene expression and/or NGPCR gene product activity. Such compounds can be used as therapeutic agents for the treatment of various symptomatic representations of biological disorders or imbalances.

### 4. DESCRIPTION OF THE SEQUENCE LISTING AND FIGURES

The Sequence Listing provides the sequences of the described NGPCR ORFs and the amino acid sequences encoded thereby. SEQ ID NO:5 describes a NGPCR ORF and flanking regions.

### 5. DETAILED DESCRIPTION OF THE INVENTION

The human NGPCRs described for the first time herein are novel receptor proteins that are expressed in human pituitary, testis, skeletal muscle, adipose, esophagus, cervix, pericardium, fetal kidney, and fetal lung cells. The described NGPCR sequences were obtained using human genomic sequences in conjunction with cDNAs generated from mRNAs from human testis, pituitary, and adipose (Edge Biosystems, Gaithersburg, MD, and Clontech, Palo Alto, CA). The described NGPCRs are transmembrane proteins of the 7TM family of receptors. As with other GPCRs, signal transduction is triggered when a ligand binds to the receptor. Interfering with the binding of the natural ligand, or neutralizing or removing the ligand, or interference with its binding to a NGPCR will effect NGPCR mediated signal transduction. Because of their biological significance, 7TM, and particularly GPCR, proteins have been subjected to intense scientific and commercial scrutiny (see, for example, U.S. Patent Nos. 5,942,416 and 5,891,720, both of which are herein incorporated by reference in their entirety, for applications, uses, and assays involving NGPCRs). In addition to 7TM proteins, the presently described NGPCRs share significant homology with GPCRs of the hepta-helical receptor families.

The invention encompasses the use of the described NGPCR nucleotides, NGPCR proteins and peptides, as well as antibodies, preferably humanized monoclonal antibodies, or binding fragments, domains, or fusion proteins thereof, to the NGPCRs (which can, for example, act as NGPCR agonists or antagonists), antagonists that inhibit receptor activity or expression, or agonists that activate receptor activity or increase its expression, in the diagnosis and treatment of disease.

In particular, the invention described in the subsections below encompasses NGPCR polypeptides or peptides corresponding to functional domains of NGPCR (*e.g*., ECD, TM or CD), mutated, truncated or deleted NGPCRs *(e.g.,* NGPCRs missing one or more functional domains or portions thereof, such as, ΔECD, ΔTM and/or ΔCD), NGPCR fusion proteins *(e.g.,* a NGPCR or a functional domain of a NGPCR, such as the ECD, fused to an unrelated protein or peptide such as an immunoglobulin constant region, *i.e*., IgFc), nucleotide sequences encoding such products, and host cell expression systems that can produce such NGPCR products.

The invention also encompasses antibodies and anti-idiotypic antibodies (including Fab fragments), antagonists and agonists of the NGPCRs, as well as compounds or nucleotide constructs that inhibit expression of a NGPCR gene (transcription factor inhibitors, antisense and ribozyme molecules, or gene or regulatory sequence replacement constructs), or promote expression of NGPCR (e.g., expression constructs in which NGPCR coding sequences are operatively associated with expression control elements such as promoters, promoter/enhancers, etc.). The invention also relates to host cells and animals genetically engineered to express the human NGPCRs (or mutants thereof) or to inhibit or "knock-out" expression of the animal's endogenous NGPCR genes.

The NGPCR proteins or peptides, NGPCR fusion proteins, NGPCR nucleotide sequences, antibodies, antagonists and agonists can be useful for the detection of mutant NGPCRs or inappropriately expressed NGPCRs for the diagnosis of disease. The NGPCR proteins or peptides, NGPCR fusion proteins, NGPCR nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of a NGPCR in the body. The use of engineered host cells and/or animals may offer an advantage in that such systems allow not only for the identification of compounds that bind to an ECD of a NGPCR, but can also identify compounds that affect the signal transduced by an activated NGPCR.

Finally, the NGPCR protein products (especially soluble derivatives such as peptides corresponding to a NGPCR ECD, or truncated polypeptides lacking one or more TM domains) and fusion protein products (especially NGPCR-Ig fusion proteins, *i.e.,* fusions of a NGPCR, or a domain of a NGPCR, *e.g.,* ECD, ΔTM to an IgFc), antibodies and anti-idiotypic antibodies (including Fab fragments), antagonists or agonists (including compounds that modulate signal transduction, which may act on downstream targets in a NGPCR-mediated signal transduction pathway) can be used for therapy of such diseases. For example, the administration of an effective amount of soluble NGPCR ECD, ΔTM, or an ECD-IgFc fusion protein or an anti-idiotypic antibody (or its Fab) that mimics the NGPCR ECD would "mop up" or "neutralize" the endogenous NGPCR ligand, and prevent or reduce binding and receptor activation. Nucleotide constructs encoding such NGPCR products can be used to genetically engineer host cells to express such products *in vivo*; these genetically engineered cells function as "bioreactors" in the body, delivering a continuous supply of a NGPCR, a NGPCR peptide, soluble ECD or ΔTM or a NGPCR fusion protein, that will "mop up" or neutralize a NGPCR ligand. Nucleotide constructs encoding functional NGPCRs, mutant NGPCRs, as well as antisense and ribozyme molecules can be used in "gene therapy" approaches for the modulation of NGPCR expression. Thus, the invention also encompasses pharmaceutical formulations and methods for treating biological disorders.

Various aspects of the invention are described in greater detail in the subsections below.

### 5.1 THE NGPCR POLYNUCLEOTIDES

The cDNA sequences and deduced amino acid sequences of the described human NGPCRs are presented in the Sequence Listing. The described NGPCRs can be expressed in a variety of human tissues, as described above, and are apparently encoded on human chromosome 6.

Several polymorphism were identified during the sequencing of the NGPCRs, including a G/A polymorphism at nucleotide position 239 of SEQ ID NO:1 (which results in a gln or arg being present at the corresponding amino acid (aa) position 80 of SEQ ID NO:2); a G/T polymorphism at nucleotide position 723 of SEQ ID NO:1 (both of which result in the same amino acid being present at the corresponding aa position of SEQ ID NO:2); an A/T polymorphism at nucleotide position 766 of SEQ ID NO:1 (which results in a leu or met being present at the corresponding aa position 256 of SEQ ID NO:2); a T/C polymorphism at nucleotide position 1074 of SEQ ID NO:1 (both of which result in the same amino acid being present at the corresponding aa position of SEQ ID NO:2); a G/A polymorphism at nucleotide position 1075 of SEQ ID NO:1 (which results in a glu or lys being present at the corresponding aa position 359 of SEQ ID NO:2); a G/A polymorphism at nucleotide position 1195 of SEQ ID NO:1 (which results in a ile or val being present at the corresponding aa position 399 of SEQ ID NO:2); a G/A polymorphism at nucleotide position 53 of SEQ ID NO:3 (which results in a gln or arg being present at the corresponding aa position 18 of SEQ ID NO:4); a G/T polymorphism at nucleotide position 537 of SEQ ID NO:3 (both of which result in the same amino acid being present at the corresponding aa position of SEQ ID NO:4); an A/T polymorphism at nucleotide position 580 of SEQ ID NO:3 (which results in a leu or met being present at the corresponding aa position 194 of SEQ ID NO:4); a T/C polymorphism at nucleotide position 888 of SEQ ID NO:3 (both of which result in the same amino acid being present at the corresponding aa position of SEQ ID NO:4); a G/A polymorphism at nucleotide position 889 of SEQ ID NO:3 (which results in a glu or lys being present at the corresponding aa position 297 of SEQ ID NO:4); a G/A polymorphism at nucleotide position 1009 of SEQ ID NO:3 (which results in a ile or val being present at the corresponding aa position 337 of SEQ ID NO:4).

The NGPCRs of the present invention include the human DNA sequences presented in the Sequence Listing (and vectors comprising the same), and additionally contemplates any nucleotide sequence encoding a contiguous and functional NGPCR open reading frame (ORF) that hybridizes to a complement of the DNA sequences presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel et *al.,* eds., 1989, Current Protocols in Molecular Biology, Vol. I, Green Publishing Associates, Inc., and John Wiley & Sons, Inc., New York, at p. 2.10.3) and encodes a functionally equivalent gene product. Additionally contemplated are any nucleotide sequences that hybridize to the complement of DNA sequences that encode and express an amino acid sequence presented in the Sequence Listing under moderately stringent conditions, e.g., washing in 0.2x SSC/0.1% SDS at 42°C (Ausubel et al., 1989, supra), yet that still encode a functionally equivalent NGPCR gene product. Functional equivalents of a NGPCR include naturally occurring NGPCRs present in other species, and mutant NGPCRs, whether naturally occurring or engineered (by site directed mutagenesis, gene shuffling, directed evolution as described in, for example, U.S. Patent Nos. 5,837,458 and 5,723,323, both of which are herein incorporated by reference in their eritirety). The invention also includes degenerate nucleic acid variants of the disclosed NGPCR polynucleotide sequences.

Additionally contemplated are polynucleotides encoding NGPCR ORFs, or their functional equivalents, encoded by polynucleotide sequences that are about 99, 95, 90, or about 85 percent similar or identical to corresponding regions of the polynucleotide sequences described in the Sequence Listing (as measured by BLAST sequence comparison analysis using, for example, the GCG sequence analysis package described herein using standard default parameters).

The invention also includes nucleic acid molecules, preferably DNA molecules, that hybridize to, and are therefore the complements of, the described NGPCR nucleotide sequences. Such hybridization conditions may be highly stringent or less highly stringent, as described herein. In instances wherein the nucleic acid molecules are deoxyoligonucleotides ("DNA oligos"), such molecules are about 16 to about 100 bases long, about 20 to about 80 bases long, or about 34 to about 45 bases long, or any variation or combination of sizes represented therein, incorporating a contiguous region of nucleotide sequence first disclosed in the present Sequence Listing, and can be used in conjunction with the polymerase chain reaction (PCR) to screen libraries, isolate clones, and prepare cloning and sequencing templates, etc.

Alternatively, such NGPCR oligonucleotides can be used as hybridization probes for screening libraries, and assessing gene expression patterns (particularly using a microarray or high-throughput "chip" format). Additionally, a series of the described NGPCR oligonucleotide sequences, or the complements thereof, can be used to represent all or a portion of the described NGPCR sequences. An oligonucleotide or polynucleotide sequence first disclosed in at least a portion of one or more of the sequences of SEQ ID NOS:1-5 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (i.e., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS:1-5, or an amino acid sequence encoded thereby. Methods for attaching biopolymers to, or synthesizing biopolymers on, solid support matrices, and conducting binding studies thereon are disclosed in, inter alia, U.S. Patent Nos. 5,700,637, 5,556,752, 5,744,305, 4,631,211, 5,445,934, 5,252,743, 4,713,326, 5,424,186, and 4,689,405, the disclosures of which are herein incorporated by reference in their entirety.

Addressable arrays comprising sequences first disclosed in SEQ ID NOS:1-5 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides and more preferably 25 nucleotides from the sequences first disclosed in SEQ ID NOS:1-5.

For example, a series of the described oligonucleotide sequences, or the complements thereof, can be used in chip format to represent all or a portion of the described NGPCRs. The oligonucleotides, typically between about 16 to about 40 (or any whole number within the stated range) nucleotides in length, can partially overlap each other and/or the NGPCR sequence may be represented using oligonucleotides that do not overlap. Accordingly, the described polynucleotide sequences shall typically comprise at least about two or three distinct oligonucleotide sequences of at least about 18 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense (3'-to-5') orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-5 provides detailed information about transcriptional changes involved in a specific pathway, potentially leading to the identification of novel components or gene functions that manifest themselves as novel phenotypes.

Probes consisting of sequences first disclosed in SEQ ID NOS:1-5 can also be used in the identification, selection and validation of novel molecular targets for drug discovery. The use of these unique sequences permits the direct confirmation of drug targets and recognition of drug dependent changes in gene expression that are modulated through pathways distinct from the intended target of the drug. These unique sequences therefore also have utility in defining and monitoring both drug action and toxicity.

As an example of utility, the sequences first disclosed in SEQ ID NOS:1-5 can be utilized in microarrays or other assay formats, to screen collections of genetic material from patients who have a particular medical condition. These investigations can also be carried out using the sequences first disclosed in SEQ ID NOS:1-5 in *silico* and by comparing previously collected genetic databases and the disclosed sequences using computer software known to those in the art.

Thus the sequences first disclosed in SEQ ID NOS:1-5 can be used to identify mutations associated with a particular disease, and also in diagnostic or prognostic assays.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in SEQ ID NOS:1-5. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (e.g., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relative to one or more additional sequence(s) or one or more restriction sites present in the disclosed sequence.

For oligonucleotides probes, highly stringent conditions may refer, e.g., to washing in 6x SSC/0.05% sodium pyrophosphate at 37°C (for 14-base oligos), 48°C (for 17-base oligos), 55°C (for 20-base oligos), and 60°C (for 23-base oligos). The described oligonucleotides may encode or act as NGPCR antisense molecules, useful, for example, in NGPCR gene regulation (and/or as antisense primers in amplification reactions of NGPCR gene nucleic acid sequences). With respect to NGPCR gene regulation, such techniques can be used to regulate biological functions. Further, such sequences may be used as part of ribozyme and/or triple helix sequences, also useful for NGPCR gene regulation.

Additionally, the antisense oligonucleotides may comprise at least one modified base moiety that is selected from the group including, but not limited to, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including, but not limited to, arabinose, 2-fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. *15*:6625-6641). The oligonucleotide is a 2'-0-methylribonucleotide (Inoue et *al.,* 1987, Nucl. Acids Res. 15:6131-6148), or a chimeric RNADNA analogue (Inoue et *al.,* 1987, FEBS Lett. *215*:327-330). Alternatively, double stranded RNA can be used to disrupt the expression and function of a targeted NGPCR.

Oligonucleotides of the invention may be synthesized by standard methods known in the art, e.g., by use of an automated DNA synthesizer (such as are commercially available from Biosearch, Applied Biosystems, etc.). As examples, phosphorothioate oligonucleotides may be synthesized (Stein et *al*., 1988, Nucl. Acids Res. 16:3209), methylphosphonate oligonucleotides can be prepared by use of controlled pore glass polymer supports (Sarin *et al.,* 1988, Proc. Natl. Acad. Sci. U.S.A. 85:7448-7451), etc.

Low stringency conditions are well-known to those of skill in the art, and will vary predictably depending on the specific organisms from which the library and the labeled sequences are derived. For guidance regarding such conditions see, for example, Sambrook *et al*., 1989, Molecular Cloning, A Laboratory Manual, Cold Spring Harbor Press, N.Y. (and periodic updates thereof); and Ausubel *et al.,* 1989, supra (and periodic updates thereof).

Alternatively, suitably labeled NGPCR nucleotide probes may be used to screen a human genomic library using appropriately stringent conditions, or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), *etc.,* that can be used in diagnostics and pharmacogenomics.

For example, the present sequences can be used in restriction fragment length polymorphism (RFLP) analysis to identify specific individuals. In this technique, an individual's genomic DNA is digested with one or more restriction enzymes, and probed on a Southern blot to yield unique bands for identification (as generally described in U.S. Patent No. 5,272,057, incorporated herein by reference). In addition, the sequences of the present invention can be used to provide polynucleotide reagents, e.g., PCR primers, targeted to specific loci in the human genome, which can enhance the reliability of DNA-based forensic identifications by, for example, providing another "identification marker" (i.e., another DNA sequence that is unique to a particular individual). Actual base sequence information can be used for identification as an accurate alternative to patterns formed by restriction enzyme generated fragments.

Further, NGPCR homologs may be isolated from nucleic acids of the organism of interest by performing PCR using two degenerate oligonucleotide primer pools designed on the basis of amino acid sequences within the NGPCR gene products disclosed herein. The template for the reaction may be total RNA, mRNA, and/or cDNA obtained by reverse transcription of mRNA prepared from, for example, human or non-human cell lines or tissue(s) known to express, or suspected of expressing, a NGPCR gene allele.

The PCR product may be subcloned and sequenced to ensure that the amplified sequences represent the sequence of the desired NGPCR gene. The PCR fragment may then be used to isolate a full length cDNA clone by a variety of methods. For example, the amplified fragment may be labeled and used to screen a cDNA library, such as a bacteriophage cDNA library. Alternatively, the labeled fragment may be used to isolate genomic clones via the screening of a genomic library.

PCR technology may also be utilized to isolate full length NGPCR cDNA sequences. For example, RNA may be isolated, following standard procedures, from an appropriate cellular or tissue source (i.e., one known to express, or suspected of expressing, a NGPCR gene). A reverse transcription (RT) reaction may be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment may easily be isolated. For a review of cloning strategies that may be used, see e.g., Sambrook et *al.,* 1989, *supra.*

A cDNA of a mutant NGPCR gene can be isolated, for example, by using PCR. In this case, the first cDNA strand may be synthesized by hybridizing an oligo-dT oligonucleotide to mRNA isolated from tissue known to express, or suspected of expressing, a mutant NGPCR allele, in an individual putatively carrying a mutant NGPCR allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well-known to those of skill in the art. By comparing the DNA sequence of the mutant NGPCR allele to that of the normal NGPCR allele, the mutation(s) responsible for the loss or alteration of function of the mutant NGPCR gene product can be ascertained.

Alternatively, a genomic library can be constructed using DNA obtained from an individual suspected of carrying, or known to carry, a mutant NGPCR allele, or a cDNA library can be constructed using RNA from a tissue known to express, or suspected of expressing, a mutant NGPCR allele. A normal NGPCR gene, or any suitable fragment thereof, can then be labeled and used as a probe to identify the corresponding mutant NGPCR allele in such libraries. Clones containing the mutant NGPCR gene sequences can then be purified and subjected to sequence analysis according to methods well-known to those of skill in the art.

Additionally, an expression library can be constructed utilizing cDNA synthesized from, for example, RNA isolated from a tissue known to express, or suspected of expressing, a mutant NGPCR allele in an individual suspected of carrying, or known to carry, such a mutant allele. In this manner, gene products made by the putatively mutant tissue may be expressed and screened using standard antibody screening techniques in conjunction with antibodies raised against the normal NGPCR gene product, as described, below, in Section 5.3 (for screening techniques, see, for example, Harlow and Lane, eds., 1988, "Antibodies: A Laboratory Manual", Cold Spring Harbor Press, Cold Spring Harbor, incorporated herein in its entirety by reference).

Additionally, screening can be accomplished by screening with labeled NGPCR fusion proteins, such as, for example, alkaline phosphatase-NGPCR or NGPCR-alkaline phosphatase fusion proteins. In cases where a NGPCR mutation results in an expressed gene product with altered function (e.g., as a result of a missense or a frameshift mutation), a polyclonal set of antibodies to NGPCR are likely to cross-react with the mutant NGPCR gene product. Library clones detected via their reaction with such labeled antibodies can be purified and subjected to sequence analysis according to methods well-known to those of skill in the art.

The invention also encompasses nucleotide sequences that encode mutant NGPCRs, peptide fragments of the NGPCRs, truncated NGPCRs, and NGPCR fusion proteins. These include, but are not limited to, nucleotide sequences encoding mutant NGPCRs described below, polypeptides or peptides corresponding to one or more ECD, TM and/or CD domains of the NGPCR or portions of these domains, truncated NGPCRs in which one or two of the domains is deleted, e.g., a soluble NGPCR lacking the TM or both the TM and CD regions, or a truncated, nonfunctional NGPCR lacking all or a portion of the CD region. Nucleotides encoding fusion proteins may include, but are not limited to, full length NGPCR sequences, truncated NGPCRs, or nucleotides encoding peptide fragments of NGPCR fused to an unrelated protein or peptide, such as, for example, a transmembrane sequence, which anchors the NGPCR ECD to the cell, an IgFc domain, which increases the stability and half life of the resulting fusion protein (e.g., NGPCR-Ig) in the bloodstream, or an enzyme, fluorescent protein, or luminescent protein that can be used as a marker.

The invention also encompasses: (a) DNA vectors that contain any of the foregoing NGPCR coding sequences and/or their complements (i.e., antisense); (b) DNA expression vectors that contain any of the foregoing NGPCR coding sequences operatively associated with at least a first regulatory element that directs the expression of the coding sequences (for example, baculovirus vectors as described in U.S. Patent No. 5,869,336 herein incorporated by reference); (c) genetically engineered host cells that contain any of the foregoing NGPCR coding sequences operatively associated with at least a first regulatory element that directs the expression of the coding sequences in the host cell; and (d) genetically engineered host cells that express an endogenous NGPCR sequence under the control of an exogenously introduced regulatory element (i.e., gene activation). As used herein, regulatory elements include, but are not limited to, inducible and non-inducible promoters, enhancers, operators and other elements known to those skilled in the art that drive and regulate expression. Such regulatory elements include, but are not limited to, the cytomegalovirus hCMV immediate early gene, regulatable, viral (particularly retroviral LTR promoters) the early or late promoters of SV40 or adenovirus, the lac system, the trp system, the tet system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

An additional application of the described NGPCR polynucleotide sequences is their use in the molecular mutagenesis/evolution of proteins that are at least partially encoded by the described novel sequences using, for example, polynucleotide shuffling or related methodologies. Such approaches are described in U.S. Patent Nos. 5,830,721, 5,723,323, and 5,837,458, which are herein incorporated by reference in their entirety.

Additionally contemplated uses for the described sequences include the engineering of constitutively "on" variants for use in cell assays and genetically engineered animals, using the methods and applications described in U.S. Provisional Patent Application Ser Nos. 60/110,906, 60/106,300, 60/094,879, and 60/121,851, each of which are herein incorporated by reference in their entirety.

NGPCR gene products can also be expressed in transgenic animals. Animals of any species, including, but not limited to, worms, mice, rats, rabbits, guinea pigs, pigs, micro-pigs, birds, goats, and non-human primates, e.g., baboons, monkeys, and chimpanzees, may be used to generate NGPCR transgenic animals.

Any technique known in the art may be used to introduce a NGPCR transgene into animals to produce the founder lines of transgenic animals. Such techniques include, but are not limited to, pronuclear microinjection (Hoppe and Wagner, 1989, U.S. Patent No. 4,873,191); retrovirus mediated gene transfer into germ lines (Van der Putten et *al*., 1985, Proc. Natl. Acad. Sci., USA 82:6148-6152); gene targeting in embryonic stem cells (Thompson et *al*., 1989, Cell 56:313-321); electroporation of embryos (Lo, 1983, Mol Cell. Biol. *3*:1803-1814); and sperm-mediated gene transfer (Lavitrano et *al.,* 1989, Cell *57*:717-723); etc. For a review of such techniques, see Gordon, 1989, Transgenic Animals, Intl. Rev. Cytol. *115*:171-229, which is incorporated by reference herein in its entirety.

The present invention provides for transgenic animals that carry the NGPCR transgene in all their cells, as well as animals that carry the transgene in some, but not all their cells, i.e., mosaic animals or somatic cell transgenic animals. The transgene may be integrated as a single transgene or in concatamers, e.g., head-to-head tandems or head-to-tail tandems. The transgene may also be selectively introduced into and activated in a particular cell type by following, for example, the teaching of Lasko *et al.,* 1992, Proc. Natl. Acad. Sci. USA 89:6232-6236. The regulatory sequences required for such a cell-type specific activation will depend upon the particular cell type of interest, and will be apparent to those of skill in the art.

When it is desired that a NGPCR transgene be integrated into the chromosomal site of the endogenous NGPCR gene, gene targeting is preferred. Briefly, when such a technique is to be utilized, vectors containing some nucleotide sequences homologous to the endogenous NGPCR gene are designed for the purpose of integrating, via homologous recombination with chromosomal sequences, into and disrupting the function of the nucleotide sequence of the endogenous NGPCR gene (i.e., "knockout" animals).

The transgene can also be selectively introduced into a particular cell type, thus inactivating the endogenous NGPCR gene in only that cell type, by following, for example, the teaching of Gu *et al.,* 1994, Science, *265*:103-106. The regulatory sequences required for such a cell-type specific inactivation will depend upon the particular cell type of interest, and will be apparent to those of skill in the art.

Once transgenic animals have been generated, the expression of the recombinant NGPCR gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques that include, but are not limited to, Northern blot analysis of tissue samples obtained from the animal, *in situ* hybridization analysis, and RT-PCR. Samples of NGPCR gene-expressing tissue may also be evaluated immunocytochemically using antibodies specific for the NGPCR transgene product.

### 5.2 NGPCR PROTEINS AND POLYPEPTIDES

NGPCR proteins, polypeptides and peptide fragments, mutated, truncated or deleted forms of the NGPCRs and/or NGPCR fusion proteins can be prepared for a variety of uses, including, but not limited to, use as protein therapeutics, the generation of antibodies, as reagents in diagnostic assays, the identification of other cellular gene products related to a NGPCR, as reagents in assays for screening for compounds that can be used as pharmaceutical reagents useful in the therapeutic treatment of mental, biological, or medical disorders (i.e., kidney disorders, improper blood pressure, etc.) and disease. The described NGPCRs share structural similarity with compounds including, but not limited to, latrotoxin receptors and latrophilins. Given the similarity information and expression data, the described NGPCRs can be targeted (by drugs, oligos, antibodies, etc.) in order to treat disease, or to therapeutically augment the efficacy of therapeutic agents.

The Sequence Listing discloses the amino acid sequences encoded by the described NGPCR nucleotide sequences. The NGPCRs have initiator methionines in DNA sequence contexts consistent with translation initiation sites, followed by hydrophobic signal sequences typical of membrane associated proteins or secreted proteins. The sequence data presented herein indicate that alternatively spliced forms of the NGPCRs exist.

The NGPCR amino acid sequences of the invention include the amino acid sequences presented in the Sequence Listing, as well as analogues and derivatives thereof. Further, corresponding NGPCR homologues from other species are encompassed by the invention. In fact, any NGPCR protein encoded by the NGPCR nucleotide sequences described above are within the scope of the invention, as are any novel polynucleotide sequences encoding all or any novel portion of an amino acid sequence presented in the Sequence Listing. The degenerate nature of the genetic code is well-known, and, accordingly, each amino acid presented in the Sequence Listing is generically representative of the well-known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, Darnell et *al.,* eds., Scientific American Books, New York, NY, herein incorporated by reference), are generically representative of all the various permutations and combinations of nucleic acid sequences that can encode such amino acid sequences.

The invention also encompasses proteins that are functionally equivalent to the NGPCRs encoded by the described nucleotide sequences as judged by any of a number of criteria, including, but not limited to, the ability to bind a ligand of a NGPCR, the ability to effect an identical or complementary signal transduction pathway, a change in cellular metabolism (e.g., ion flux, tyrosine phosphorylation, etc.), or a change in phenotype when the NGPCR equivalent is present in an appropriate cell type (such as the amelioration, prevention or delay of a biochemical, biophysical, or overt phenotype). Such functionally equivalent NGPCR proteins include, but are not limited to, additions or substitutions of amino acid residues within the amino acid sequences encoded by the NGPCR nucleotide sequences described above, but that result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

While random mutations can be made to a NGPCR DNA (using random mutagenesis techniques well-known to those skilled in the art), and the resulting mutant NGPCRs tested for activity, site-directed mutations of the NGPCR coding sequence can be engineered (using site-directed mutagenesis techniques well-known to those skilled in the art) to generate mutant NGPCRs with increased function (e.g., higher binding affinity for the target ligand and/or greater signaling capacity) or decreased function (e.g., weaker binding affinity for the target ligand and/or decreased signal transduction capacity). One starting point for such analysis is by aligning the disclosed human sequences with corresponding gene/protein sequences from other mammals, for example, in order to identify amino acid sequence motifs that are conserved between different species. Non-conservative changes can be engineered at variable positions to alter function, signal transduction capability, or both. Alternatively, where alteration of function is desired, deletion or non-conservative alterations of the conserved regions (i.e., identical amino acids) can be engineered, for example, deletion or non-conservative alterations (substitutions or insertions) of the various conserved transmembrane domains.

Other mutations in the NGPCR coding sequence can be made to generate NGPCRs that are better suited for expression, scale up, etc., in the host cells chosen. For example, cysteine residues can be deleted or substituted with another amino acid in order to eliminate disulfide bridges, and N-linked glycosylation sites can be altered or eliminated to achieve, for example, expression of a homogeneous product that is more easily recovered and purified from yeast hosts, which are known to hyperglycosylate N-linked sites. To this end, a variety of amino acid substitutions at one or both of the first or third amino acid positions of any one or more of the glycosylation recognition sequences that occur in the ECD (N-X-S or N-X-T), and/or an amino acid deletion at the second position of any one or more such recognition sequences in the ECD, will prevent glycosylation of a NGPCR at the modified tripeptide sequence (see, e.g., Miyajima et *al*., 1986, EMBO J. *5*:1193-1197).

Peptides corresponding to one or more domains of the NGPCR *(e.g.,* ECD, TM, CD, *etc.),* truncated or deleted NGPCRs *(e.g.,* NGPCR in which a ECD, TM and/or CD is deleted), as well as fusion proteins in which a full length NGPCR, a NGPCR peptide, or truncated NGPCR is fused to an unrelated protein, are also within the scope of the invention, and can be designed on the basis of the presently disclosed NGPCR nucleotide and NGPCR amino acid sequences. Such fusion proteins include, but are not limited to: IgFc fusions, which stabilize the NGPCR protein or peptide and prolong half-life *in vivo;* fusions to any amino acid sequence that allows the fusion protein to be anchored to the cell membrane, allowing an ECD to be exhibited on the cell surface; or fusions to an enzyme, fluorescent protein, or luminescent protein that provides a marker function.

While the NGPCR polypeptides and peptides can be chemically synthesized (e.g., see Creighton, 1983, Proteins: Structures and Molecular Principles, W.H. Freeman & Co., N.Y.), large polypeptides derived from a NGPCR and full length NGPCRs can be advantageously produced by recombinant DNA technology using techniques well-known in the art for expressing nucleic acid containing NGPCR gene sequences and/or coding sequences. Such methods can be used to construct expression vectors containing one or more of the presently described NGPCR nucleotide sequences and appropriate transcriptional and translational control signals. These methods include, for example, *in vitro* recombinant DNA techniques, synthetic techniques, and *in vivo* genetic recombination (see, for example, the techniques described in Sambrook *et al*., 1989, *supra,* and Ausubel *et al.,* 1989, supra). Alternatively, RNA corresponding to all or a portion of a transcript encoded by a NGPCR nucleotide sequence may be chemically synthesized using, for example, synthesizers (see, for example, the techniques described in "Oligonucleotide Synthesis", 1984, Gait, M.J., ed., IRL Press, Oxford, which is incorporated by reference herein in its entirety).

A variety of host-expression vector systems may be utilized to express the NGPCR nucleotide sequences of the invention. Where the NGPCR peptide or polypeptide is a soluble derivative (e.g., NGPCR peptides corresponding to an ECD; truncated or deleted NGPCR in which a TM and/or CD are deleted), the peptide or polypeptide can be recovered from the culture, i.e., from the host cell in cases where the NGPCR peptide or polypeptide is not secreted, and from the culture media in cases where the NGPCR peptide or polypeptide is secreted by the host cell. However, such expression systems also encompass engineered host cells that express a NGPCR, or functional equivalent, *in situ, i.e.,* anchored in the cell membrane. Purification or enrichment of NGPCR from such expression systems can be accomplished using appropriate detergents and lipid micelles and methods well-known to those skilled in the art. However, such engineered host cells themselves may be used in situations where it is important not only to retain the structural and functional characteristics of the NGPCR, but to assess biological activity, *e.g.*, in drug screening assays.

The expression systems that may be used for purposes of the invention include, but are not limited to, microorganisms such as bacteria *(e.g., E. coli, B. subtilis)* transformed with recombinant bacteriophage DNA, plasmid DNA or cosmid DNA expression vectors containing NGPCR nucleotide sequences; yeast *(e.g., Saccharomyces, Pichia)* transformed with recombinant yeast expression vectors containing NGPCR nucleotide sequences; insect cell systems infected with recombinant virus expression vectors *(e.g.,* baculovirus) containing NGPCR nucleotide sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing NGPCR nucleotide sequences; or mammalian cell systems (e.g., COS, CHO, BHK, 293, 3T3) harboring recombinant expression constructs containing NGPCR nucleotide sequences and promoters derived from the genome of mammalian cells (e.g., metallothionein promoter) or from mammalian viruses (e.g., the adenovirus late promoter or the vaccinia virus 7.5K promoter).

In bacterial systems, a number of expression vectors may be advantageously selected depending upon the use intended for the NGPCR gene product being expressed. For example, when a large quantity of such a protein is to be produced, such as in the generation of pharmaceutical compositions of NGPCR protein or for raising antibodies to a NGPCR protein, vectors that direct the expression of high levels of fusion protein products that are readily purified may be desirable. Such vectors include, but are not limited, to the *E*. *coli* expression vector pUR278 (Ruther et *al*., 1983, EMBO J. 2:1791), in which a NGPCR coding sequence may be ligated individually into the vector in frame with the *lacZ* coding region so that a fusion protein is produced; pIN vectors (Inouye and Inouye, 1985, Nucleic Acids Res. 13:3101-3109; Van Heeke and Schuster, 1989, J. Biol. Chem. *264*:5503-5509); and the like. pGEX vectors may also be used to express NGPCR proteins, polypeptides or peptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the NGPCR protein, polypeptide or peptide can be released from the GST moiety.

In an exemplary insect system, *Autographa californica* nuclear polyhedrosis virus (AcNPV) is used as a vector to express NGPCR nucleotide sequences. The virus grows in *Spodoptera frugiperda* cells. A NGPCR gene coding sequence may be cloned individually into a non-essential region (for example the polyhedrin gene) of the virus and placed under control of an AcNPV promoter (for example the polyhedrin promoter). Successful insertion of NGPCR coding sequence will result in inactivation of the polyhedrin gene and production of non-occluded recombinant virus *(i.e.,* virus lacking the proteinaceous coat coded for by the polyhedrin gene). These recombinant viruses are then used to infect *Spodoptera frugiperda* cells in which the inserted gene is expressed *(e.g.,* see Smith et al., 1983, J. Virol. 46:584; Smith, U.S. Patent No. 4,215,051).

In mammalian host cells, a number of viral-based expression systems may be utilized. In cases where an adenovirus is used as an expression vector, a NGPCR nucleotide sequence of interest may be ligated to an adenovirus transcription/translation control complex, e.g., the late promoter and tripartite leader sequence. This chimeric sequence may then be inserted in the adenovirus genome by *in vitro* or *in vivo* recombination. Insertion in a non-essential region of the viral genome (e.g., region E1 or E3) will result in a recombinant virus that is viable and capable of expressing a NGPCR gene product in infected hosts (e.g., see Logan and Shenk, 1984, Proc. Natl. Acad. Sci. USA *81*:3655-3659). Specific initiation signals may also be required for efficient translation of inserted NGPCR nucleotide sequences. These signals include the ATG initiation codon and adjacent sequences. In cases where an entire NGPCR gene or cDNA, including its own initiation codon and adjacent sequences, is inserted into an appropriate expression vector, no additional translational control signals may be needed. However, in cases where only a portion of a NGPCR coding sequence is inserted, exogenous translational control signals, including, perhaps, the ATG initiation codon, may be provided. Furthermore, the initiation codon should be in phase with the reading frame of the desired NGPCR coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bitter *et al*., 1987, Methods in Enzymol. 153:516-544).

In addition, a host cell strain may be chosen that modulates the expression of the inserted sequences, or modifies and processes the expression product, in the specific fashion desired. Such modifications *(e.g.,* glycosylation) and processing (e.g., cleavage) of protein products may be important for the function of the protein. Different host cells have characteristic and specific mechanisms for the post-translational processing and modification of proteins and gene or expression products. Appropriate cell lines or host systems can be chosen to ensure the correct modification and processing of the foreign protein expressed. To this end, eukaryotic host cells that possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, and WI38 cell lines.

For long-term, high-yield production of recombinant proteins, stable expression is preferred. For example, cell lines that stably express the NGPCR sequences described above may be engineered. Rather than using expression vectors that contain viral origins of replication, host cells can be transformed with DNA controlled by appropriate expression control elements (e.g., promoter, enhancer sequences, transcription terminators; polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci, which in turn can be cloned and expanded into cell lines. This method may advantageously be used to engineer cell lines that express a NGPCR gene product. Such engineered cell lines may be particularly useful in screening and evaluation of compounds that affect the endogenous activity of a NGPCR gene product.

A number of selection systems can be used, including, but not limited to, the herpes simplex virus thymidine kinase (Wigler *et al.,* 1977, Cell *11*:223), hypoxanthine-guanine phosphoribosyltransferase (Szybalska and Szybalski, 1962, Proc. Natl. Acad. Sci. USA 48:2026), and adenine phosphoribosyltransferase (Lowy *et al.,* 1980, Cell *22*:817) genes, which can be employed in tk-, hgprt- or aprt⁻ cells, respectively. Also, antimetabolite resistance can be used as the basis of selection for the following genes: dhfr, which confers resistance to methotrexate (Wigler et *al*., 1980, Natl. Acad. Sci. USA 77:3567; O'Hare *et al.,* 1981, Proc. Natl. Acad. Sci. USA 78:1527); gpt, which confers resistance to mycophenolic acid (Mulligan and Berg, 1981, Proc. Natl. Acad. Sci. USA 78:2072); neo, which confers resistance to the aminoglycoside G-418 (Colberre-Garapin *et al*., 1981, J. Mol. Biol. 150:1); and hygro, which confers resistance to hygromycin (Santerre et *al*., 1984, Gene 30:147).

Alternatively, any fusion protein can be readily purified by utilizing an antibody specific for the fusion protein being expressed. For example, one such system allows for the ready purification of non-denatured fusion proteins expressed in human cell lines (Janknecht *et al.,* 1991, Proc. Natl. Acad. Sci. USA 88:8972-8976). In this system, the gene of interest is subcloned into a vaccinia recombination plasmid such that the open reading frame is translationally fused to an amino-terminal tag consisting of six histidine residues. Extracts from cells infected with recombinant vaccinia virus are loaded onto Ni²⁺·nitriloacetic acid-agarose columns, and histidine-tagged proteins are selectively eluted with imidazole-containing buffers.

Also encompassed by the present invention are fusion proteins that direct a NGPCR to a target organ and/or facilitate transport across the membrane into the cytosol. Conjugation of NGPCRs to antibody molecules, or their Fab fragments, could be used to target cells bearing a particular epitope. Attaching an appropriate signal sequence to a NGPCR would also transport the NGPCR to a desired location within the cell. Alternatively, targeting of NGPCRs or their nucleic acid sequences might be achieved using liposomes or lipid complex based delivery systems. Such technologies are described in "Liposomes: A Practical Approach", New, R.R.C., ed., Oxford University Press, New York, and in U.S. Patent Nos. 4,594,595, 5,459,127, 5,948,767 and 6,110,490 and their respective disclosures, which are herein incorporated by reference in their entirety. Additionally embodied are novel protein constructs engineered in such a way that they facilitate transport of a NGPCR to a target site or desired organ, where the NGPCR crosses the cell membrane and/or the nucleus and exerts its functional activity. This goal may be achieved by coupling of a NGPCR to a cytokine or other ligand that provides targeting specificity, and/or to a protein transducing domain (see generally U.S. Patent Application Ser. Nos. 60/111,701 and 60/056,713, both of which are herein incorporated by reference, for examples of such transducing sequences), to facilitate passage across cellular membranes, and/or can optionally be engineered to include one or more nuclear localization signal(s).

### 5.3 ANTIBODIES TO NGPCR PROTEINS

Antibodies that specifically recognize one or more epitopes of a NGPCR, or epitopes of conserved variants of a NGPCR, or peptide fragments of a NGPCR, are also encompassed by the invention. Such antibodies include, but are not limited to, polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NGPCRs in a biological sample and may, therefore, be utilized as part of a diagnostic or prognostic technique whereby patients may be tested for abnormal amounts of one or more NGPCR. Such antibodies may also be utilized in conjunction with, for example, compound screening schemes, as described below, for the evaluation of the effect of test compounds on expression and/or activity of a NGPCR expression or gene product. Additionally, such antibodies can be used in conjunction with gene therapy to, for example, evaluate the normal and/or engineered NGPCR-expressing cells prior to their introduction into a patient. Such antibodies may additionally be used as a method for the inhibition of abnormal NGPCR activity. Thus, such antibodies may be utilized as part of a variety of therapeutic regimens, such as, for example, in weight disorder treatment methods.

For the production of antibodies, various host animals may be immunized by injection with a NGPCR, one or more NGPCR peptide (e.g., one corresponding to a functional domain of the receptor, such as an ECD, TM or CD), truncated NGPCR polypeptide(s) (NGPCR in which one or more domains, e.g., a TM or CD, has been deleted), functional equivalents of a NGPCR, or mutants of a NGPCR. Such host animals may include, but are not limited to, rabbits, mice, and rats, to name but a few. Various adjuvants may be used to increase the immunological response, depending on the host species, including, but not limited to, Freund's adjuvant (complete and incomplete), mineral salts such as aluminum hydroxide or aluminum phosphate, chitosan, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and/or coupling with molecules such as keyhole limpet hemocyanin, tetanus toxoid, diphtheria toxoid, ovalbumin, cholera toxin, or fragments thereof. Polyclonal antibodies are heterogeneous populations of antibody molecules derived from the sera of the immunized animals.

Monoclonal antibodies, which are homogeneous populations of antibodies to a particular antigen, may be obtained by any technique that provides for the production of antibody molecules by continuous cell lines in culture. These include, but are not limited to, the hybridoma technique (Kohler and Milstein, 1975, Nature *256*:495-497; and U.S. Patent No. 4,376,110), the human B-cell hybridoma technique (Kosbor *et al.,* 1983, Immunology Today 4:72; Cole et *al*., 1983, Proc. Natl. Acad. Sci. USA 80:2026-2030), and the EBV-hybridoma technique (Cole *et al*., 1985, Monoclonal Antibodies And Cancer Therapy, Alan R. Liss, Inc., pp. 77-96). Such antibodies may be of any immunoglobulin class, including IgG, IgM, IgE, IgA, IgD, and any subclass thereof. The hybridoma producing the mAbs of this invention may be cultivated *in vitro* or *in vivo.* Production of high titers of mAbs *in vivo* makes this the presently preferred method of production.

In addition, techniques developed for the production of "chimeric antibodies" (Morrison *et al*., 1984, Proc. Natl. Acad. Sci., *81*:6851-6855; Neuberger *et al*., 1984, Nature, *312*:604-608; Takeda *et al*., 1985., Nature, *314*:452-454), by splicing the genes from a mouse antibody molecule of appropriate antigen specificity together with genes from a human antibody molecule of appropriate biological activity, can be used. A chimeric antibody is a molecule in which different portions are derived from different animal species, such as those having a variable region derived from a murine mAb and a human immunoglobulin constant region. Such technologies are described in U.S. Patent Nos. 6,075,181 and 5,877,397 and their respective disclosures, which are herein incorporated by reference in their entirety. Also encompassed by the present invention is the use of fully humanized monoclonal antibodies, as described in U.S. Patent No. 6,150,584 and respective disclosures, which are herein incorporated by reference in their entirety.

Alternatively, techniques described for the production of single chain antibodies (U.S. Patent No. 4,946,778; Bird, 1988, Science *242*:423-426; Huston *et al*., 1988, Proc. Natl. Acad. Sci. USA 85:5879-5883; and Ward et *al.,* 1989, Nature 341:544-546) can be adapted to produce single chain antibodies against NGPCR peptides, polypeptides and/or proteins. Single chain antibodies are formed by linking the heavy and light chain fragments of the Fv region via an amino acid bridge, resulting in a single chain polypeptide.

Antibody fragments that recognize specific epitopes may be generated by known techniques. For example, such fragments include, but are not limited to: F(ab')₂ fragments, which can be produced by pepsin digestion of an antibody molecule; and Fab fragments, which can be generated by reducing the disulfide bridges of F(ab')₂ fragments. Alternatively, Fab expression libraries may be constructed (Huse *et al.,* 1989, Science, 246:1275-1281) to allow rapid and easy identification of monoclonal Fab fragments with the desired specificity.

Antibodies to a NGPCR can, in turn, be utilized to generate anti-idiotype antibodies that "mimic" a given NGPCR, using techniques well-known to those skilled in the art (see, e.g., Greenspan and Bona, 1993, FASEB J 7:437-444; and Nissinoff, 1991, J. Immunol. 147:2429-2438). For example antibodies that bind to a NGPCR ECD and competitively inhibit the binding of a ligand of NGPCR can be used to generate anti-idiotypes that "mimic" a NGPCR ECD and, therefore, bind and neutralize a ligand. Such neutralizing anti-idiotypes, or Fab fragments of such anti-idiotypes, can be used in therapeutic regimens involving the NGPCR signaling pathway.

Additionally given the high degree of relatedness of mammalian NGPCRs, the presently described knock-out mice (having never seen a particular NGPCR, and thus never been tolerized to the NGPCR), have a unique utility, as they can be advantageously applied to the generation of antibodies against the disclosed mammalian NGPCRs (*i.e*., NGPCRs will be immunogenic in NGPCR knock-out animals).

### 5.4 DIAGNOSIS OF ABNORMALITIES RELATED TO A NGPCR

A variety of methods can be employed for the diagnostic and prognostic evaluation of disorders related to NGPCR function, and for the identification of subjects having a predisposition to such disorders.

Such methods can, for example, utilize reagents such as the NGPCR nucleotide sequences described in Section 5.1, and/or NGPCR antibodies, as described, in Section 5.3. Specifically, such reagents may be used, for example, for: (1) the detection of the presence of NGPCR gene mutations, or the detection of either over- or under-expression of NGPCR mRNA relative to a given (i.e., normal) phenotype; (2) the detection of either an over- or an under-abundance of NGPCR gene product relative to a given (i.e., normal) phenotype; and (3) the detection of perturbations or abnormalities in the signal transduction pathway mediated by NGPCRs.

The methods described herein may be performed, for example, by utilizing pre-packaged diagnostic kits comprising at least one specific NGPCR nucleotide sequence and/or NGPCR antibody reagent described herein, which may be conveniently used, *e.g*., in clinical settings to diagnose patients exhibiting medical disorders or abnormalities, such as, for example, body weight disorder abnormalities.

For the detection of NGPCR mutations, any nucleated cell can be used as a starting source for genomic nucleic acid. For the detection of NGPCR gene expression or NGPCR gene products, any cell type or tissue in which a NGPCR gene is expressed can be utilized.

Nucleic acid-based detection techniques and peptide detection techniques are described in greater detail below.

### 5.4.1 DETECTION OF NGPCR GENES AND TRANSCRIPTS

Mutations within a NGPCR gene or nucleotide sequence can be detected by utilizing a number of techniques. Nucleic acids from any nucleated cell can be used as the starting point for such assay techniques, and may be isolated according to standard nucleic acid preparation procedures that are well-known to those of skill in the art.

DNA may be used in hybridization or amplification assays of biological samples to detect abnormalities involving NGPCR gene structure, including point mutations, insertions, deletions and chromosomal rearrangements. Such assays may include, but are not limited to, Southern analyses, single stranded conformational polymorphism analyses (SSCP), restriction fragment length polymorphisms (RFLP, as generally described in U.S. Patent No. 5,272,057, incorporated herein by reference), coding single nucleotide polymorphisms (cSNP), and PCR analyses.

Such diagnostic methods for the detection of NGPCR gene-specific mutations can involve, for example, contacting and incubating nucleic acids, including recombinant DNA molecules, cloned genes or degenerate variants thereof, obtained from a sample, e.g., derived from a patient sample or other appropriate cellular source, with one or more labeled nucleic acid reagents, including recombinant DNA molecules, cloned genes or degenerate variants thereof, as described in Section 5.1, under conditions favorable for the specific annealing of these reagents to their complementary sequences within a given NGPCR gene or sequence. Preferably, the lengths of these nucleic acid reagents are at least 15 to 30 nucleotides. After incubation, all non-annealed nucleic acids are removed from the nucleic acid:NGPCR molecule hybrid. The presence of nucleic acids that have hybridized, if any such molecules exist, is then detected. In conjunction with such a detection scheme, the nucleic acid from the cell type or tissue of interest can be immobilized, for example, to a solid support such as a membrane, or a plastic surface such as that on a microtiter plate or polystyrene beads. In such a case, after incubation, non-annealed, labeled nucleic acid reagents of the type described in Section 5.1 are easily removed. Detection of the remaining, annealed, labeled NGPCR nucleic acid reagents is accomplished using standard techniques well-known to those in the art. The NGPCR sequences to which the nucleic acid reagents have annealed can be compared to the annealing pattern expected from a normal NGPCR sequence in order to determine whether a NGPCR mutation is present.

Alternative diagnostic methods for the detection of NGPCR gene specific nucleic acid molecules, in patient samples or other appropriate cell sources, may involve their amplification, e.g., by PCR (the experimental embodiment set forth in U.S. Patent Nos. 4,683,195; 4,683,202 and 4,800,159, which are incorporated herein by reference in their entirety), followed by the detection of the amplified molecules using techniques well-known to those of skill in the art. The resulting amplified sequences can be compared to those that would be expected if the nucleic acid being amplified contained only normal copies of a NGPCR gene in order to determine whether a NGPCR gene mutation exists.

Additionally, well-known genotyping techniques can be performed to identify individuals carrying NGPCR gene mutations. Such techniques include, for example, the use of restriction fragment length polymorphisms (RFLPs), which involve sequence variations in one of the recognition sites for the specific restriction enzyme used.

Additionally, improved methods for analyzing DNA polymorphisms that can be utilized for the identification of NGPCR gene mutations have been described, which capitalize on the presence of variable numbers of short, tandemly repeated DNA sequences between restriction enzyme sites. For example, Weber (U.S. Patent No. 5,075,217, which is incorporated.herein by reference in its entirety) describes a DNA marker based on length polymorphisms in blocks of (dC-dA)n-(dG-dT)n short tandem repeats. The average separation of (dC-dA)n-(dG-dT)n blocks is estimated to be 30,000-60,000 bp. Markers that are so closely spaced exhibit a high frequency co-inheritance, and are extremely useful in the identification of genetic mutations, such as, for example, mutations within a given NGPCR gene, and the diagnosis of diseases and disorders related to NGPCR mutations.

Also, Caskey et al. (U.S. Patent No. 5,364,759, which is incorporated herein by reference in its entirety) describe a DNA profiling assay for detecting short tri- and tetra-nucleotide repeat sequences. The process includes extracting the DNA of interest, such as the NGPCR gene, amplifying the extracted DNA, and labeling the repeat sequences to form a genotypic map of the individual's DNA.

The level of NGPCR gene expression can also be assayed by detecting and measuring NGPCR transcription. For example, RNA from a cell type or tissue known to express, or suspected of expressing, a NGPCR gene can be isolated and tested utilizing hybridization or PCR techniques such as those described herein. The isolated cells can be derived from cell culture or from a patient. The analysis of cells taken from culture may be a necessary step in the assessment of cells to be used as part of a cell-based gene therapy technique or, alternatively, to test the effect of compounds on the expression of a NGPCR gene. Such analyses may reveal both quantitative and qualitative aspects of the expression pattern of a NGPCR gene, including activation or inactivation of NGPCR gene expression.

In one embodiment of such a detection scheme, cDNAs are synthesized from the RNAs of interest *(e.g.,* by reverse transcription of the RNA molecule into cDNA). A sequence within the cDNA is then used as the template for a nucleic acid amplification reaction, such as a PCR amplification reaction, or the like. The nucleic acid reagents used as synthesis initiation reagents (*e.g*., primers) in the reverse transcription and nucleic acid amplification steps of this method are chosen from among the NGPCR nucleic acid reagents described in Section 5.1. The preferred lengths of such nucleic acid reagents are at least 9-30 nucleotides. For detection of the amplified product, the nucleic acid amplification may be performed using radioactively or non-radioactively labeled nucleotides. Alternatively, enough amplified product may be made such that the product may be visualized by standard ethidium bromide staining or any other suitable nucleic acid staining method, or by sequencing.

Additionally, it is possible to perform such NGPCR gene expression assays "in situ", *i.e*., directly upon tissue sections (fixed and/or frozen) of patient tissue obtained from biopsies or resections, such that no nucleic acid purification is necessary. Nucleic acid reagents, such as those described above, may be used as probes and/or primers for such in *situ* procedures (see, for example, Nuovo, 1992, "PCR *In Situ* Hybridization: Protocols And Applications", Raven Press, NY).

Alternatively, if a sufficient quantity of the appropriate cells can be obtained, standard Northern analysis can be performed to determine the level of NGPCR mRNA expression.

Additionally, NGPCR oligonucleotide or polynucleotide sequences can be used as hybridization probes in conjunction with a solid support matrix/substrate (*e.g*., resins, beads, membranes, plastics, polymers, metal or metallized substrates, gene chips, and crystalline or polycrystalline substrates, etc.).

### 5.4.2 DETECTION OF NGPCR GENE PRODUCTS

Antibodies directed against wild-type or mutant NGPCR gene products, or conserved variants or peptide fragments thereof, which are discussed above, may also be used as diagnostics and prognostics, as described herein. Such diagnostic methods may be used to detect abnormalities in the level of NGPCR gene expression, or abnormalities in the structure and/or temporal, tissue, cellular, or subcellular location of a NGPCR, and may be performed *in vivo* or *in vitro*, such as, for example, on biopsy tissue.

For example, antibodies directed to epitopes of a NGPCR ECD can be used *in vivo* to detect the pattern and level of expression of the particular NGPCR in the body. Such antibodies can be labeled, *e.g*., with a radio-opaque or other appropriate compound, and injected into a subject in order to visualize binding to NGPCRs expressed in the body, using methods such as X-rays, CAT-scans, or MRI. Labeled antibody fragments, *e.g*., the Fab or single chain antibody comprising the smallest portion of the antigen binding region, are preferred for this purpose, in order to promote crossing the blood-brain barrier and permit labeling of NGPCRs expressed in the brain.

Additionally, any NGPCR fusion protein or NGPCR conjugated protein, whose presence can be detected, can be administered. For example, NGPCR fusion or conjugated proteins labeled with a radio-opaque or other appropriate compound can be administered and visualized *in vivo*, as discussed above for labeled antibodies. Further, such NGPCR fusion proteins as alkaline phosphatase-NGPCR on NGPCR-alkaline phosphatase fusion proteins can be utilized for *in vitro* diagnostic procedures.

Alternatively, immunoassays or fusion protein detection assays, as described above, can be utilized on biopsy and autopsy samples *in vitro* to permit assessment of the expression pattern of a NGPCR. Such assays are not confined to the use of antibodies that define a NGPCR ECD, but can include the use of antibodies directed to epitopes of any of the domains of a NGPCR, *e.g.*, the ECD, the TM and/or CD. The use of each or all of these labeled antibodies will yield useful information regarding translation and intracellular transport of a NGPCR to the cell surface, and can identify defects in processing.

The tissue or cell type to be analyzed will generally include those that are known to express, or suspected of expressing, a NGPCR gene. The protein isolation methods employed herein may, for example, be such as those previously described (Harlow and Lane, 1988, supra). The isolated cells can be derived from cell culture or from a patient. The analysis of cells taken from culture may be a necessary step in the assessment of cells that could be used as part of a cell-based gene therapy technique or, alternatively, to test the effect of compounds on the expression of a NGPCR gene.

For example, antibodies, or fragments of antibodies, such as those described above in Section 5.3 as useful in the present invention, may be used to quantitatively or qualitatively detect the presence of NGPCR gene products, or conserved variants or peptide fragments thereof. This can be accomplished, for example, by immunofluorescence techniques employing a fluorescently labeled antibody (see below, this Section), coupled with light microscopic, flow cytometric, or fluorimetric detection. Such techniques are especially preferred if such NGPCR gene products are expressed on the cell surface.

The antibodies (or fragments thereof), or NGPCR fusion or conjugated proteins, useful in the present invention may, additionally, be employed histologically, as in immunofluorescence, immunoelectron microscopy or non-immuno assays, for *in situ* detection of NGPCR gene products or conserved variants or peptide fragments thereof, or for NGPCR binding (in the case of labeled NGPCR ligand fusion proteins).

*In situ* detection may be accomplished by removing a histological specimen from a patient, and applying thereto a labeled antibody or fusion protein of the present invention. The antibody (or fragment) or fusion protein is preferably applied by overlaying the labeled antibody (or fragment) onto a biological sample. Through the use of such a procedure, it is possible to determine not only the presence of a NGPCR gene product, or conserved variants or peptide fragments, or NGPCR binding, but also NGPCR distribution in the examined tissue. Using the present invention, those of ordinary skill will readily perceive that any of a wide variety of histological methods (such as staining procedures) can be modified in order to achieve such *in situ* detection.

Immunoassays and non-immunoassays for NGPCR gene products, or conserved variants or peptide fragments thereof, will typically comprise incubating a sample, such as a biological fluid, a tissue extract, freshly harvested cells, or lysates of cells that have been incubated in cell culture, in the presence of a detectably labeled antibody capable of identifying NGPCR gene products, or conserved variants or peptide fragments thereof, and detecting the bound antibody by any of a number of techniques well-known in the art.

The biological sample may be brought in contact with and immobilized onto a solid phase support or carrier, such as nitrocellulose or any other solid support that is capable of immobilizing cells, cell particles or soluble proteins. The support may then be washed with suitable buffers followed by treatment with the detectably labeled NGPCR antibody or NGPCR ligand fusion protein. The solid phase support may then be washed with the buffer a second time to remove unbound antibody or fusion protein. The amount of bound label remaining on the solid support may then be detected by conventional means.

By "solid phase support or carrier" is intended any support capable of binding an antigen or an antibody. Well-known supports or carriers include, but are not limited to, glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material can have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding an antibody or antigen, or will be able to ascertain the same by use of routine experimentation.

The binding activity of a given lot of NGPCR antibody or NGPCR ligand fusion protein may be determined according to well-known methods. Those skilled in the art will be able to determine operative and optimal assay conditions for each determination by employing routine experimentation.

With respect to antibodies, one of the ways in which a NGPCR antibody can be detectably labeled is by linking it to an enzyme for use in an enzyme immunoassay (EIA) (Voller, 1978, Diagnostic Horizons 2:1-7, Microbiological Associates Quarterly Publication, Walkersville, MD; Voller et *al*., 1978, J. Clin. Pathol. 31:507-520; Butler, 1981, Meth. Enzymol. 73:482-523; Maggio, E. (ed.), 1980, Enzyme Immunoassay, CRC Press, Boca Raton, FL,; and Ishikawa et *al*., (eds.), 1981, Enzyme Immunoassay, Kgaku Shoin, Tokyo). The enzyme that is bound to the antibody will react with an appropriate substrate, preferably a chromogenic substrate, in such a manner as to produce a chemical moiety that can be detected, for example, by spectrophotometric, fluorimetric or by visual means. Enzymes that can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods, which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect a NGPCR through the use of a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a gamma counter, a scintillation counter, or by autoradiography.

It is also possible to label the antibody with a fluorescent compound. When the fluorescently labeled antibody is exposed to light of the proper wavelength, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibodies (or fragments thereof) of the present invention. Bioluminescence is a type of chemiluminescence found in biological systems, in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin (green fluorescent protein and mutants thereof; as described in U. S. Patent Nos. 5,491,084, 5,625,048, 5,777,079, 5,795,737, 5,804,387, 5,874,304, 5,968,750, 5,976,796, 6,020,192, 6,027,882, 6,054,321, 6,096,865, 6,146,826, 6,172,188 and 6,265,548, each of which is hereby incorporated by reference).

### 5.5 SCREENING ASSAYS FOR COMPOUNDS THAT MODULATE NGPCR EXPRESSION OR ACTIVITY

The following assays are designed to identify: compounds that interact with (e.g., bind to) NGPCRs (including, but not limited to, an ECD or CD of a NGPCR); intracellular proteins that interact with a NGPCR (including, but not limited to, the TM and CD of NGPCR); compounds that interfere with the interaction of NGPCR with transmembrane or intracellular proteins involved in NGPCR-mediated signal transduction; and compounds that modulate the activity of a NGPCR gene *(i.e.,* modulate the level of NGPCR gene expression) or modulate the level of NGPCR. Assays may additionally be utilized that identify compounds that bind to NGPCR gene regulatory sequences (e.g., promoter sequences), and that may modulate NGPCR gene expression (see e.g., Platt, 1994, J. Biol. Chem. 269:28558-28562, which is incorporated herein by reference in its entirety).

The compounds that can be screened in accordance with the invention include, but are not limited to, peptides, antibodies and fragments thereof, and other organic compounds (e.g., peptidomimetics) that bind to an ECD of a NGPCR and either mimic the activity triggered by the natural ligand (i.e., agonists) or inhibit the activity triggered by the natural ligand (i.e., antagonists); as well as peptides, antibodies or fragments thereof, and other organic compounds that mimic the ECD of the NGPCR (or a portion thereof) and bind to and "neutralize" the natural ligand.

Such compounds may include, but are not limited to, peptides, such as, for example, soluble peptides, including, but not limited to, members of random peptide libraries (see, e.g., Lam et *al*., 1991, Nature 354:82-84; Houghten et *al*., 1991, Nature 354:84-86), and combinatorial chemistry-derived molecular libraries made of D- and/or L- configuration amino acids, phosphopeptides (including, but not limited to, members of random or partially degenerate, directed phosphopeptide libraries; see, e.g., Songyang et *al*., 1993, Cell 72:767-778), antibodies (including, but not limited to, polyclonal, monoclonal, humanized, anti-idiotypic, chimeric or single chain antibodies, and Fab, F(ab')₂ and Fab expression library fragments, and epitope-binding fragments thereof), and small organic or inorganic molecules.

Other compounds that can be screened in accordance with the invention include, but are not limited to, small organic molecules that are able to cross the blood-brain barrier, gain entry into an appropriate cell (e.g., in the cerebellum, the hypothalamus, etc.), and affect the expression of a NGPCR gene or some other gene involved in the NGPCR signal transduction pathway (e.g., by interacting with the regulatory region or transcription factors involved in gene expression); or such compounds that affect the activity of a NGPCR (e.g., by inhibiting or enhancing the enzymatic activity of a CD) or the activity of some other intracellular factor involved in the NGPCR signal transduction pathway.

Computer modeling and searching technologies permit identification of compounds, or the improvement of already identified compounds, that can modulate NGPCR expression or activity. Having identified such a compound or composition, the active sites or regions are identified. Such active sites might typically be ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site is determined. This can be done by known methods, including X-ray crystallography, which can determine a complete molecular structure. On the other hand, solid or liquid phase NMR can be used to determine certain intra-molecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by such modeling methods.

Finally, having determined the structure of the active site, either experimentally, by modeling, or by a combination thereof, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. The compounds found from such a search are potential NGPCR modulating compounds.

Alternatively, these methods can be used to identify improved modulating compounds from an already known modulating compound or ligand. The composition of the known compound can be modified, and the structural effects of modification can be determined using the experimental and computer modeling methods described above applied to the new composition. The altered structure is then compared to the active site structure of the compound to determine if an improved fit or interaction results. In this manner systematic variations in composition, such as by varying side groups, can be quickly evaluated to obtain modified modulating compounds or ligands of improved specificity or activity.

Further experimental and computer modeling methods useful to identify modulating compounds based upon identification of the active sites of a NGPCR, and related transduction and transcription factors, will be apparent to those of skill in the art.

Examples of molecular modeling systems are the CHARMM and QUANTA programs (Polygen Corporation, Waltham, MA). CHARMM performs the energy minimization and molecular dynamics functions, while QUANTA performs the construction, graphic modeling and analysis of molecular structure. QUANTA allows interactive construction, modification, visualization, and analysis of the behavior of molecules with each other.

A number of articles review computer modeling of drugs interactive with specific proteins, such as: Rotivinen et *al.,* 1988, Acta Pharmaceutical Fennica 97:159-166; Ripka, New Scientist 54-57 (June 16, 1988); McKinaly and Rossmann, 1989, Annu. Rev. Pharmacol. Toxiciol. *29*:111-122; Perry and Davies, OSAR: Quantitative Structure-Activity Relationships in Drug Design, pp. 189-193 (Alan R. Liss, Inc. 1989); Lewis and Dean, 1989 Proc. R. Soc. Lond. 236:125-140 and 141-162; and, with respect to a model receptor for nucleic acid components, Askew et *al.,* 1989, J. Am. Chem. Soc. *111*:1082-1090. Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario). Although these are primarily designed for application to drugs specific to particular proteins, they can be adapted to design of drugs specific to NGPCRs, or regions of NGPCR DNA or RNA, once that region is identified.

Although described above with reference to design and generation of compounds that could alter binding, one could also screen libraries of known compounds, including natural products or synthetic chemicals, and biologically active materials, including proteins, for compounds that are inhibitors or activators.

Cell-based systems can also be used to identify compounds that bind NGPCRs, as well as assess the altered activity associated with such binding in living cells. One tool of particular interest for such assays is green fluorescent protein, which is described, inter *alia,* in U.S. Patent No. 5,625,048, herein incorporated by reference. Cells that may be used in such cellular assays include, but are not limited to, leukocytes, or cell lines derived from leukocytes, lymphocytes, stem cells, including embryonic stem cells, and the like. In addition, expression host cells (e.g., B95 cells, COS cells, CHO cells, OMK cells, fibroblasts, Sf9 cells) genetically engineered to express a functional NGPCR of interest and to respond to activation by the test, or natural, ligand, as measured by a chemical or phenotypic change, or induction of another host cell gene, can be used as an end point in the assay.

### 5.5.1 IN VITRO SCREENING ASSAYS FOR COMPOUNDS THAT BIND TO NGPCRs

*In vitro* systems can be designed to identify compounds capable of interacting with (e.g., binding to) a NGPCR (including, but not limited to, a ECD or CD of a NGPCR). Compounds identified may be useful, for example, in modulating the activity of wild-type and/or mutant NGPCR gene products, or in elaborating the biological function of the NGPCR; may be utilized in screens for identifying compounds that disrupt normal NGPCR interactions; or may in themselves disrupt such interactions.

The principle of the assays used to identify compounds that bind to a NGPCR involves preparing a reaction mixture of a NGPCR and a test compound under conditions and for a time sufficient to allow the two components to interact and bind, thus forming a complex that can be removed and/or detected in the reaction mixture. The NGPCR species used can vary depending upon the goal of the screening assay. For example, where agonists of the natural ligand are sought, the full length NGPCR, or a soluble truncated NGPCR, e.g., in which the TM and/or CD is deleted from the molecule, a peptide corresponding to an ECD, or a fusion protein containing one or more NGPCR ECD fused to a protein or polypeptide that affords advantages in the assay system (e.g., labeling, isolation of the resulting complex, etc.), can be utilized. Where compounds that interact with the cytoplasmic domain are sought to be identified, peptides corresponding to a NGPCR CD or fusion proteins containing a NGPCR CD can be used.

The screening assays can be conducted in a variety of ways. For example, one method to conduct such an assay would involve anchoring the NGPCR protein, polypeptide, peptide, or fusion protein, or the test substance, onto a solid phase and detecting NGPCR/test compound complexes anchored on the solid phase at the end of the reaction. In one embodiment of such a method, the NGPCR reactant may be anchored onto a solid surface, and the test compound, which is not anchored, may be labeled, either directly or indirectly. Examples of some of the technologies available to immobilize the molecules are discussed in Cass, ed., "Immobilized Biomolecules In Analysis: A Practical Approach", Oxford University Press, NY.

In practice, microtiter plates may conveniently be utilized as the solid phase. The anchored component may be immobilized by non-covalent or covalent attachments. Non-covalent attachment may be accomplished by simply coating the solid surface with a solution of the protein and drying. Alternatively, an immobilized antibody, preferably a monoclonal antibody, specific for the protein to be immobilized may be used to anchor the protein to the solid surface. The surfaces may be prepared in advance and stored.

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. where the previously non-immobilized component is pre-labeled, the detection of label immobilized on the surface indicates that complexes were formed. Where the previously non-immobilized component is not pre-labeled, an indirect label can be used to detect complexes anchored on the surface, e.g., using a labeled antibody specific for the previously non-immobilized component (the antibody, in turn, may be directly labeled or indirectly labeled with a labeled anti-Ig antibody).

Alternatively, a reaction can be conducted in a liquid phase, the reaction products separated from unreacted components, and complexes detected, e.g., using an immobilized antibody specific for a NGPCR protein, polypeptide, peptide, or fusion protein, or the test compound, to anchor any complexes formed in solution, and a labeled antibody specific for the other component of the possible complex to detect anchored complexes.

Alternatively, cell-based assays can be used to identify compounds that interact with a NGPCR. To this end, cell lines that express a NGPCR, or cell lines (e.g., COS cells, CHO cells, fibroblasts, etc.) that have been genetically engineered to express a NGPCR *(e.g.,* by transfection or transduction of NGPCR DNA) can be used. Interaction of the test compound with, for example, a ECD of a NGPCR expressed by the host cell can be determined by comparison or competition with native ligand.

### 5.5.2. ASSAYS FOR INTRACELLULAR PROTEINS THAT INTERACT WITH NGPCRs

Any method suitable for detecting protein-protein interactions may be employed for identifying transmembrane proteins or intracellular proteins that interact with a NGPCR. Among the traditional methods that may be employed are co-immunoprecipitation, crosslinking and co-purification through gradients or chromatographic columns of cell lysates, or proteins obtained from cell lysates, and a NGPCR to identify proteins in the lysate that interact with the NGPCR. For these assays, the NGPCR component used can be a full length NGPCR, a soluble derivative lacking the membrane-anchoring region (e.g., a truncated NGPCR in which a TM is deleted resulting in a truncated molecule containing a ECD fused to a CD), a peptide corresponding to a CD, or a fusion protein containing a CD of a NGPCR. Once isolated, such an intracellular protein can be identified and can, in turn, be used in conjunction with standard techniques to identify proteins with which it interacts. For example, at least a portion of the amino acid sequence of an intracellular protein that interacts with a NGPCR can be ascertained using techniques well-known to those of skill in the art, such as via the Edman degradation technique (see, e.g., Creighton, 1983, "Proteins: Structures and Molecular Principles", W.H. Freeman & Co., N.Y., pp.34-49). The amino acid sequence obtained may be used as a guide for the generation of oligonucleotide mixtures that can be used to screen for nucleotide sequences encoding such intracellular proteins. Screening can be accomplished, for example, by standard hybridization or PCR techniques. Techniques for the generation of oligonucleotide mixtures and the screening are well-known (see, e.g., Ausubel, supra, and Innis et *al*., eds., PCR Protocols: A Guide to Methods and Applications, 1990, Academic Press, Inc., New York).

Additionally, methods may be employed that result in the simultaneous identification of genes that encode transmembrane or intracellular proteins that interact with a NGPCR. These methods include, for example, probing expression libraries, in a manner similar to the well-known technique of antibody probing of λgt11 libraries, using labeled NGPCR protein, or an NGPCR polypeptide, peptide or fusion protein, e.g., an NGPCR polypeptide or NGPCR domain fused to a marker (e.g., an enzyme, fluor, luminescent protein, or dye), or an Ig-Fc domain.

One method that detects protein interactions *in vivo,* the two-hybrid system, is described in detail for illustration only, and not by way of limitation. One version of this system utilizes yeast cells (Chien et *al.,* 1991, Proc. Natl. Acad. Sci. USA, *88*:9578-9582), while another uses mammalian cells (Luo et *al*., 1997, Biotechniques, 22:350-352). Both the yeast and mammalian two-hybrid systems are commercially available from Clontech (Palo Alto, CA), and are further described in U.S. Patent Nos. 5,283,173; 5,468,614, and 5,667,973, which are herein incorporated by reference in their entirety.

Briefly, utilizing such a system, plasmids are constructed that encode two hybrid proteins: one plasmid consists of nucleotides encoding the DNA-binding domain of a transcription activator protein fused to a NGPCR nucleotide sequence encoding a NGPCR protein, polypeptide, peptide, or fusion protein, and the other plasmid consists of nucleotides encoding an activation domain of a transcription activator protein fused to a cDNA encoding an unknown protein to be tested for interaction with a NGPCR, which has been recombined into this plasmid as part of a cDNA library. The DNA-binding domain fusion plasmid and the cDNA library are transformed into a strain of the yeast *Saccharomyces cerevisiae* or a mammalian cell (such as Saos-2, CHO, CV1, Jurkat or HeLa) that contains a reporter gene (e.g., HBS, lacZ, CAT, or a gene encoding an essential amino acid) whose regulatory region contains the binding site of the transcription activator. Either hybrid protein alone cannot activate transcription of the reporter gene: the DNA-binding domain hybrid cannot because it does not provide activation function; and the activation domain hybrid cannot because it cannot localize to the binding site of the activator. Interaction of the two hybrid proteins reconstitutes the functional activator protein and results in expression of the reporter gene, which is detected by an assay for the reporter gene product.

The two-hybrid system, or related methodologies, may be used to screen activation domain libraries for proteins that interact with the "bait" gene product. By way of example, and not by way of limitation, a NGPCR may be used as the bait gene product. Total genomic or cDNA sequences are fused to DNA encoding an activation domain. This library and a plasmid encoding a hybrid of a bait NGPCR gene product fused to the DNA-binding domain are co-transformed into a reporter strain, and the resulting transformants are screened for those that express the reporter gene. For example, and not by way of limitation, a bait NGPCR gene sequence, such as an open reading frame of a NGPCR (or a domain of a NGPCR), can be cloned into a vector such that it is translationally fused to DNA encoding the DNA-binding domain of the GAL4 protein. These colonies are purified and the library plasmids responsible for reporter gene expression are isolated. DNA sequencing is then used to identify the proteins encoded by the library plasmids.

A cDNA library of the cell line from which proteins that interact with bait NGPCR gene product are to be detected can be made using methods routinely practiced in the art. According to one particular system, for example, the cDNA fragments can be inserted into a vector such that they are translationally fused to the transcriptional activation domain of GAL4. This library can be co-transformed along with the bait NGPCR gene-GAL4 fusion plasmid into a yeast strain that: a) cannot grow without added histidine; and b) contains a HIS3 gene driven by a promoter that contains GAL4 activation sequence. A cDNA encoded protein, fused to a GAL4 transcriptional activation domain, which interacts with bait MARK3 gene product will reconstitute an active GAL4 protein, and thereby drive expression of the HIS3 gene. Colonies that express HIS3 can be detected by growth on petri dishes containing semi-solid agar based media lacking histidine. The cDNA can then be purified from these strains, and used to produce and isolate the bait NGPCR gene-interacting protein using techniques routinely practiced in the art.

### 5.5.3. ASSAYS FOR COMPOUNDS THAT INTERFERE WITH NGPCR/INTRACELLULAR OR NGPCR/TRANSMEMBRANE MACROMOLECULE INTERACTION

The macromolecules that interact with a NGPCR are referred to, for purposes of this discussion, as "binding partners." These binding partners are likely to be involved in a NGPCR signal transduction pathway. Therefore, it is desirable to identify compounds that interfere with or disrupt the interaction of such binding partners with NGPCRs, which compounds may be useful in regulating the activity of a NGPCR and controlling disorders associated with NGPCR activity. For example, given their expression pattern, the described NGPCRs are contemplated to be particularly useful in methods for identifying compounds useful in the therapeutic treatment of high or low blood pressure (and associated symptoms), kidney disorders, weight control disorders, metabolic disorders, and cancer.

The basic principle of the assay systems used to identify compounds that interfere with the interaction between a NGPCR and its binding partner or partners involves preparing a reaction mixture containing a NGPCR protein, polypeptide, peptide, or fusion protein, as described in Sections 5.5.1 and 5.5.2 above, and the binding partner under conditions and for a time sufficient to allow the two components to interact and bind, thus forming a complex. In order to test a compound for inhibitory activity, the reaction mixture is prepared in the presence and absence of the test compound. The test compound may be initially included in the reaction mixture, or may be added at a time subsequent to the addition of the NGPCR moiety and its binding partner. Control reaction mixtures are incubated without the test compound or with a placebo. The formation of any complexes between the NGPCR moiety and the binding partner is then detected. The formation of a complex in the control reaction, but not in the reaction mixture containing the test compound, indicates that the compound interferes with the interaction of the NGPCR and the interactive binding partner. Additionally, complex formation within reaction mixtures containing the test compound and a normal NGPCR protein may also be compared to complex formation within reaction mixtures containing the test compound and a mutant NGPCR. This comparison may be important in those cases wherein it is desirable to identify compounds that specifically disrupt interactions of mutant, or mutated, NGPCRs, but not normal NGPCRs.

The assay for compounds that interfere with the interaction of a NGPCR and its binding partner(s) can be conducted in a heterogeneous or homogeneous format. Heterogeneous assays involve anchoring either the NGPCR moiety or the binding partner onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction by competition can be identified by conducting the reaction in the presence of the test substance, i.e., by adding the test substance to the reaction mixture prior to, or simultaneously with, a NGPCR moiety and interactive binding partner. Alternatively, test compounds that disrupt preformed complexes, e.g., compounds with higher binding constants that displace one of the components from the complex, can be tested by adding the test compound to the reaction mixture after complexes have been formed. The various formats are described briefly below.

In a heterogeneous assay system, either a NGPCR moiety or an interactive binding partner is anchored onto a solid surface, while the non-anchored species is labeled, either directly or indirectly. In practice, microtiter plates are conveniently utilized. The anchored species may be immobilized by non-covalent or covalent attachments. Non-covalent attachment may be accomplished simply by coating the solid surface with a solution of a NGPCR gene product or binding partner and drying. Alternatively, an immobilized antibody specific for the species to be anchored may be used to anchor the species to the solid surface. The surfaces may be prepared in advance and stored..

In order to conduct the assay, the partner of the immobilized species is exposed to the coated surface with or without the test compound. After the reaction is complete, unreacted components are removed (e.g., by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labeled, an indirect label can be used to detect complexes anchored on the surface, e.g., using a labeled antibody specific for the initially non-immobilized species (the antibody, in turn, may be directly labeled or indirectly labeled with a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected, e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. In this approach, a preformed complex of a NGPCR moiety and an interactive binding partner is prepared in which either the NGPCR or its binding partners is labeled, but the signal generated by the label is quenched due to formation of the complex (see, *e.g.,* U.S. Patent No. 4,109,496, which utilizes this approach for immunoassays). The addition of a test substance that competes with and displaces one of the species from the preformed complex will result in the generation of a signal above background. In this way, test substances that disrupt NGPCR/intracellular binding partner interactions can be identified.

In a particular embodiment, a NGPCR fusion protein can be prepared for immobilization. For example, a NGPCR or a peptide fragment, e.g., corresponding to a CD, can be fused to a glutathione-S-transferase (GST) gene using a fusion vector, such as pGEX-5X-1, in such a manner that its binding activity is maintained in the resulting fusion protein. The interactive binding partner can be purified and used to raise a monoclonal antibody, using methods routinely practiced in the art and described above, in Section 5.3. This antibody can be labeled with a radioactive isotope, ¹²⁵I for example, by methods routinely practiced in the art. In a heterogeneous assay, e.g., the GST-NGPCR fusion protein can be anchored to glutathione-agarose beads. The interactive binding partner can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between a NGPCR gene product and the interactive binding partner can be detected by measuring the amount of radioactivity that remains associated with the glutathione-agarose beads. A successful inhibition of the interaction by the test compound will result in a decrease in measured radioactivity.

Alternatively, the GST-NGPCR fusion protein and the interactive binding partner can be mixed together in liquid in the absence of the solid glutathione-agarose beads. The test compound can be added either during or after the species are allowed to interact. This mixture can then be added to the glutathione-agarose beads, and unbound material is washed away. Again the extent of inhibition of the NGPCR/binding partner interaction can be detected by adding a labeled antibody, and measuring the radioactivity associated with the beads.

In another embodiment of the invention, these same techniques can be employed using peptide fragments that correspond to the binding domains of a NGPCR and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding one of the proteins and screening for disruption of binding in a co-immunoprecipitation assay. Compensatory mutation(s) in the sequence encoding the second species in the complex can then be selected. Sequence analysis of the genes encoding the respective proteins will reveal the mutations that correspond to the region of the protein involved in interactive binding. Alternatively, one protein can be anchored to a solid surface using methods described above, and allowed to interact with and bind to its labeled binding partner, which has been treated with a proteolytic enzyme, such as trypsin. After washing, a relatively short, labeled peptide comprising the binding domain may remain associated with the solid material, which can be isolated and identified by amino acid sequencing. Also, once the gene coding for the intracellular binding partner is obtained, short gene segments can be engineered to express peptide fragments of the protein, which can then be tested for binding activity and purified or synthesized.

For example, and not by way of limitation, a NGPCR gene product can be anchored to a solid material, as described above, by making a GST-NGPCR fusion protein and allowing it to bind to glutathione-agarose beads. The interactive binding partner can be labeled with a radioactive isotope, such as ³⁵S, and cleaved with a proteolytic enzyme, such as trypsin. Cleavage products can then be added to the anchored GST-NGPCR fusion protein and allowed to bind. After washing away unbound peptides, labeled bound material, representing the intracellular binding partner binding domain, can be eluted, purified, and analyzed for amino acid sequence by well-known methods. Peptides so identified can be produced synthetically or fused to appropriate facilitative proteins using recombinant DNA technology.

### 5.6 PHARMACEUTICAL COMPOSITIONS

Compounds identified via assays such as those described herein may be useful, for example, in elaborating the biological function of a NGPCR gene product. Such compounds can be administered to a patient at therapeutically effective doses to treat any of a variety of physiological or mental disorders. A therapeutically effective dose refers to that amount of the compound sufficient to result in any delay in onset, or any amelioration, impediment, prevention, or alteration of any biological or overt symptom.

### 5.6.1 EFFECTIVE DOSE

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index, and can be expressed as the ratio LD₅₀/ED₅₀. Compounds that exhibit large therapeutic indices are preferred. While compounds that exhibit toxic side effects may be used in certain embodiments, care should be taken to design a delivery system that targets such compounds to the site of affected tissue, in order to minimize potential damage to uninfected cells and, thereby, reduce side effects.

The data obtained from the cell culture assays and animal studies can be used in formulating a range of dosages for use in humans. The dosage of such compounds lies preferably within a range of circulating concentrations that include the ED₅₀ with little or no toxicity. The dosage may vary within this range depending upon the dosage form employed and the route of administration utilized. For any compound used in the methods of the invention, the therapeutically effective dose can be estimated initially from cell culture assays. A dose may be formulated in animal models to achieve a circulating plasma concentration range that includes the IC₅₀ (*i.e*., the concentration of the test compound that achieves a half-maximal inhibition of symptoms) as determined in cell culture. Such information can be used to more accurately determine useful doses in humans. Levels in plasma may be measured, for example, by high performance liquid chromatography.

When the therapeutic treatment of disease is contemplated, the appropriate dosage may also be determined using animal studies to determine the maximal tolerable dose, or MTD, of a bioactive agent per kilogram weight of the test subject. In general, at least one animal species tested is mammalian. Those skilled in the art regularly extrapolate doses for efficacy and avoiding toxicity to other species, including humans. Before human studies of efficacy are undertaken, Phase I clinical studies in normal subjects help establish safe doses.

Additionally, the bioactive agent may be complexed with a variety of well established compounds or structures that, for instance, enhance the stability of the bioactive agent or otherwise enhance its pharmacological properties (*e.g.*, increase *in vivo* half-life, reduce toxicity, etc.).

The therapeutic agents will be administered by any number of methods known to those of ordinary skill in the art including, but not limited to, inhalation, subcutaneous (sub-q), intravenous (I.V.), intraperitoneal (I.P.), intramuscular (I.M.) or intrathecal injection, or topically applied (transderm, ointments, creams, salves, eye drops, and the like).

### 5.6.2 FORMULATIONS AND USE

Pharmaceutical compositions for use in accordance with the present invention may be formulated in a conventional manner using one or more physiologically acceptable carriers or excipients. Thus, the compounds and their physiologically acceptable salts and solvates may be formulated for administration by inhalation or insufflation (either through the mouth or the nose) or oral, buccal, parenteral, intracranial, topical, intrathecal, or rectal administration.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose) ; fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well-known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

The compounds may be formulated for parenteral administration by injection, e.g., by bolus injection, or continuous infusion. Formulations for injection may be presented in unit dosage form, e.g., in ampoules or in multidose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device that may contain one or more unit dosage forms containing the active ingredient. The pack may, for example, comprise metal or plastic foil, such as a blister pack. The pack or dispenser device may be accompanied by instructions for administration.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein, will become apparent to those skilled in the art from the foregoing description. Such modifications are intended to fall within the scope of the appended claims. All referenced publications, patents, and patent applications are herein incorporated by reference.

## Claims

1. An isolated nucleic acid molecule comprising a nucleotide sequence that comprises at least 24 contiguous nucleotides from SEQ ID NO:1.

2. An isolated nucleic acid molecule comprising a nucleotide sequence that:
(a) encodes the amino acid sequence shown in SEQ ID NO:2; and
(b) hybridizes under stringent conditions to the nucleotide sequence of SEQ ID NO:1 or the complement thereof.

3. An isolated nucleic acid molecule according to Claim 1, wherein said molecule is a cDNA.

4. An isolated nucleic acid molecule comprising a nucleotide sequence that encodes the amino acid sequence drawn from the group consisting of SEQ ID NO:2 and SEQ ID NO:4.

5. An isolated expression vector comprising the nucleotide sequence drawn from the group consisting of SEQ ID NO:1 and SEQ ID NO:3.

6. A polypeptide encoded by a nucleic acid as claimed in any one of claims 1 to 4.

7. An antibody that binds to a polypeptide as claimed in claim 6, or a fragment or a derivative of said antibody, for example, a polyclonal or monoclonal antibody, a single chain antibody, a Fab fragment, a F(ab')2, a fragment produced by a Fab expression library, or an anti-idiotypic (anti-Id) antibody, or an epitope binding fragment of any of said antibodies or fragments.

8. A host cell that comprises a nucleic acid as claimed in any one of claims 1 to 4 or an expression vector as claimed in claim 5 and is capable of expressing the polypeptide encoded by said nucleic acid or said expression vector, optionally on the surface of said host cell.

9. An animal that is capable of expressing a nucleic acid as claimed in any one of claims 1 to 4, or that is an optionally conditional "knock-out" animal that does not express a polypeptide as claimed in claim 6.

10. Use of a nucleic acid as claimed in any one of claims 1 to 4, an expression vector as claimed in claim 5,a polypeptide as claimed in claim 6, an antibody as claimed in claim 7, a host cell as claimed in claim 8 or an animal as claimed in claim 9, in a method for screening compounds for the ability to modulate the expression of a nucleic acid as claimed in any one of claims 1 to 4 or a polypeptide as claimed in claim 6, or that modulates the activity of a polypeptide as claimed in claim 6, for example, for high throughput screening of combinatorial libraries.


Sequence Listing

<110> LEXICON GENETICS INCORPORATED

<120> Novel Human 7TM Proteins and Polynucleotides Encoding the Same

<130> 9369(1) EP/JSvn/TJH

<150> US 60/243,948
<151> 2000-10-27

<150> US 60/244,291
<151> 2000-10-30

<160> 5

<170> FastSEQ for Windows Version 4.0

<210> 1
<211> 1923
<212> DNA
<213> homo sapiens

<400> 1
   atgactcata tacttttgct gtactacttg gtgtttcttt tgcccacaga gtcctgtagg 60
   acattgtatc aggctgcaag caaaagcaag gagaaggtgc ctgccaggcc acacggtgta 120
   tgcgatggtg tctgtacaga ctacccccag tgtactcaac cttgccctcc agacactcag 180
   ggaaatatgg ggttttcatg caggcaaaag acatggcaca agatcactga cacctgccag 240
   actcttaatg ccctcaacat ctttgaggag gattcacgtt tggttcagcc atttgaagac 300
   aatataaaaa taagtgtata tactggaaag tctgagacca taacagatat gttgctacaa 360
   aagtgtccca cagatctgtc ttgtgtaatt agaaacattc agcagtctcc ctggatacca 420
   ggaaacattg ccgtaattgt gcagctctta cacaacatat caacagcaat atggacaggt 480
   gttgatgagg caaagatgca gagttacagc accatagcca accacattct taacagcaaa 540
   agcatctcca actggacttt cattcctgac agaaacagca gctatatcct gctacattca 600
   gtcaactcct ttgcaagaag gctattcata gataaacatc ctgttgacat atcagatgtc 660
   ttcattcata ctatgggcac caccatatct ggagataaca ttggaaaaaa tttcactttt 720
   tctatgagaa ttaatgatac cagcaatgaa gtcactggga gagtgttgat cagcagagat 780
   gaacttcgga aggtgccttc cccttctcag gtcatcagca ttgcatttcc aactattggg 840
   gctattttgg aagccagtct tttggaaaat gttactgtaa atgggcttgt cctgtctgcc 900
   attttgccca aggaacttaa aagaatctca ctgatttttg aaaagatcag caagtcagag 960
   gagaggagga cacagtgtgt tggctggcac tctgtggaga acagatggga ccagcaggcc 1020
   tgcaaaatga ttcaagaaaa ctcccagcaa gctgtttgca aatgtaggcc aagtgaattg 1080
   tttacctctt tctcaattct tatgtcacct cacatcttag agagtctgat tctgacttac 1140
   atcacatatg taggcctggg catttctatt tgcagcctga tcctttgctt gtccattgag 1200
   gtcctagtct ggagccaagt gacaaagaca gagatcacct atttacgcca tgtgtgcatt 1260
   gttaacattg cagccacttt gctgatggca gatgtgtggt tcattgtggc ttcctttctt 1320
   agtggcccaa taacacacca caagggatgt gtggcagcca cattttttgt tcatttcttt 1380
   tacctttctg tatttttctg gatgcttgcc aaggcactcc ttatcctcta tggaatcatg 1440
   attgttttcc ataccttgcc caagtcagtc ctggtggcat ctctgttttc agtgggctat 1500
   ggatgccctt tggccattgc tgccatcact gttgctgcca ctgaacctgg caaaggctat 1560
   ctacgacctg agatctgctg gctcaactgg gacatgacca aagccctcct ggccttcgtg 1620
   atcccagctt tggccatcgt ggtagtaaac ctgatcacag tcacactggt gattgtcaag 1680
   acccagcgag ctgccattgg caattccatg ttccaggaag tgagagccat tgtgagaatc 1740
   agcaagaaca tcgccatcct cacaccactt ctgggactga cctggggatt tggagtagcc 1800
   actgtcatcg atgacagatc cctggccttc cacattatct tctccctgct caatgcattc 1860
   caggtaagtc cagatgcttc tgaccaagtg caaagtgaga gaattcatga agatgttctg 1920
   tga 1923

<210> 2
<211> 640
<212> PRT
<213> homo sapiens

<400> 2
Met Thr His Ile Leu Leu Leu Tyr Tyr Leu Val Phe Leu Leu Pro Thr
1               5                   10                  15
Glu Ser Cys Arg Thr Leu Tyr Gln Ala Ala Ser Lys Ser Lys Glu Lys
            20                  25                  30
Val Pro Ala Arg Pro His Gly Val Cys Asp Gly Val Cys Thr Asp Tyr

        35                  40                  45
Pro Gln Cys Thr Gln Pro Cys Pro Pro Asp Thr Gln Gly Asn Met Gly
    50                  55                  60
Phe Ser Cys Arg Gln Lys Thr Trp His Lys Ile Thr Asp Thr Cys Gln
65                  70                  75                  80
Thr Leu Asn Ala Leu Asn Ile Phe Glu Glu Asp Ser Arg Leu Val Gln
                85                  90                  95
Pro Phe Glu Asp Asn Ile Lys Ile Ser Val Tyr Thr Gly Lys Ser Glu
            100                 105                 110
Thr Ile Thr Asp Met Leu Leu Gln Lys Cys Pro Thr Asp Leu Ser Cys
        115                 120                 125
Val Ile Arg Asn Ile Gln Gln Ser Pro Trp Ile Pro Gly Asn Ile Ala
    130                 135                 140
Val Ile Val Gln Leu Leu His Asn Ile Ser Thr Ala Ile Trp Thr Gly
145                 150                 155                 160
Val Asp Glu Ala Lys Met Gln Ser Tyr Ser Thr Ile Ala Asn His Ile
                165                 170                 175
Leu Asn Ser Lys Ser Ile Ser Asn Trp Thr Phe Ile Pro Asp Arg Asn
            180                 185                 190
Ser Ser Tyr Ile Leu Leu His Ser Val Asn Ser Phe Ala Arg Arg Leu
        195                 200                 205
Phe Ile Asp Lys His Pro Val Asp Ile Ser Asp Val Phe Ile His Thr
    210                 215                 220
Met Gly Thr Thr Ile Ser Gly Asp Asn Ile Gly Lys Asn Phe Thr Phe
225                 230                 235                 240
Ser Met Arg Ile Asn Asp Thr Ser Asn Glu Val Thr Gly Arg Val Leu
                245                 250                 255
Ile Ser Arg Asp Glu Leu Arg Lys Val Pro Ser Pro Ser Gln Val Ile
            260                 265                 270
Ser Ile Ala Phe Pro Thr Ile Gly Ala Ile Leu Glu Ala Ser Leu Leu
        275                 280                 285
Glu Asn Val Thr Val Asn Gly Leu Val Leu Ser Ala Ile Leu Pro Lys
    290                 295                 300
Glu Leu Lys Arg Ile Ser Leu Ile Phe Glu Lys Ile Ser Lys Ser Glu
305                 310                 315                 320
Glu Arg Arg Thr Gln Cys Val Gly Trp His Ser Val Glu Asn Arg Trp
                325                 330                 335
Asp Gln Gln Ala Cys Lys Met Ile Gln Glu Asn Ser Gln Gln Ala Val
            340                 345                 350
Cys Lys Cys Arg Pro Ser Glu Leu Phe Thr Ser Phe Ser Ile Leu Met
        355                 360                 365
Ser Pro His Ile Leu Glu Ser Leu Ile Leu Thr Tyr Ile Thr Tyr Val
    370                 375                 380
Gly Leu Gly Ile Ser Ile Cys Ser Leu Ile Leu Cys Leu Ser Ile Glu
385                 390                 395                 400
Val Leu Val Trp Ser Gln Val Thr Lys Thr Glu Ile Thr Tyr Leu Arg
                405                 410                 415
His Val Cys Ile Val Asn Ile Ala Ala Thr Leu Leu Met Ala Asp Val
            420                 425                 430
Trp Phe Ile Val Ala Ser Phe Leu Ser Gly Pro Ile Thr His His Lys
        435                 440                 445
Gly Cys Val Ala Ala Thr Phe Phe Val His Phe Phe Tyr Leu Ser Val
    450                 455                 460
Phe Phe Trp Met Leu Ala Lys Ala Leu Leu Ile Leu Tyr Gly Ile Met
465                 470                 475                 480
Ile Val Phe His Thr Leu Pro Lys Ser Val Leu Val Ala Ser Leu Phe
                485                 490                 495
Ser Val Gly Tyr Gly Cys Pro Leu Ala Ile Ala Ala Ile Thr Val Ala
            500                 505                 510
Ala Thr Glu Pro Gly Lys Gly Tyr Leu Arg Pro Glu Ile Cys Trp Leu
        515                 520                 525
Asn Trp Asp Met Thr Lys Ala Leu Leu Ala Phe Val Ile Pro Ala Leu
    530                 535                 540
Ala Ile Val Val Val Asn Leu Ile Thr Val Thr Leu Val Ile Val Lys
545                 550                 555                 560
Thr Gln Arg Ala Ala Ile Gly Asn Ser Met Phe Gln Glu Val Arg Ala
                565                 570                 575
Ile Val Arg Ile Ser Lys Asn Ile Ala Ile Leu Thr Pro Leu Leu Gly

580                 585                 590
Leu Thr Trp Gly Phe Gly Val Ala Thr Val Ile Asp Asp Arg Ser Leu
        595                 600                 605
Ala Phe His Ile Ile Phe Ser Leu Leu Asn Ala Phe Gln Val Ser Pro
    610                 615                 620
Asp Ala Ser Asp Gln Val Gln Ser Glu Arg Ile His Glu Asp Val Leu
625                 630                 635                 640

<210> 3
<211> 1737
<212> DNA
<213> homo sapiens

<400> 3
atgggtttt catgcaggca aaagacatgg cacaagatca ctgacacctg ccagactctt      60
aatgccctca acatctttga ggaggattca cgtttggttc agccatttga agacaatata     120
aaaataagtg tatatactgg aaagtctgag accataacag atatgttgct acaaaagtgt     180
cccacagatc tgtcttgtgt aattagaaac attcagcagt ctccctggat accaggaaac     240
attgccgtaa ttgtgcagct cttacacaac atatcaacag caatatggac aggtgttgat     300
gaggcaaaga tgcagagtta cagcaccata gccaaccaca ttcttaacag caaaagcatc     360
tccaactgga ctttcattcc tgacagaaac agcagctata tcctgctaca ttcagtcaac     420
tcctttgcaa gaaggctatt catagataaa catcctgttg acatatcaga tgtcttcatt     480
catactatgg gcaccaccat atctggagat aacattggaa aaaatttcac tttttctatg     540
agaattaatg ataccagcaa tgaagtcact gggagagtgt tgatcagcag agatgaactt     600
cggaaggtgc cttccccttc tcaggtcatc agcattgcat ttccaactat tggggctatt     660
ttggaagcca gtcttttgga aaatgttact gtaaatgggc ttgtcctgtc tgccattttg     720
cccaaggaac ttaaaagaat ctcactgatt tttgaaaaga tcagcaagtc agaggagagg     780
aggacacagt gtgttggctg gcactctgtg gagaacagat gggaccagca ggcctgcaaa     840
atgattcaag aaaactccca gcaagctgtt tgcaaatgta ggccaagtga attgtttacc     900
tctttctcaa ttcttatgtc acctcacatc ttagagagtc tgattctgac ttacatcaca     960
tatgtaggcc tgggcatttc tatttgcagc ctgatccttt gcttgtccat tgaggtccta    1020
gtctggagcc aagtgacaaa gacagagatc acctatttac gccatgtgtg cattgttaac    1080
attgcagcca ctttgctgat ggcagatgtg tggttcattg tggcttcctt tcttagtggc    1140
ccaataacac accacaaggg atgtgtggca gccacatttt ttgttcattt cttttacctt    1200
tctgtatttt tctggatgct tgccaaggca ctccttatcc tctatggaat catgattgtt    1260
ttccatacct tgcccaagtc agtcctggtg gcatctctgt tttcagtggg ctatggatgc    1320
cctttggcca ttgctgccat cactgttgct gccactgaac ctggcaaagg ctatctacga    1380
cctgagatct gctggctcaa ctgggacatg accaaagccc tcctggcctt cgtgatccca    1440
gctttggcca tcgtggtagt aaacctgatc acagtcacac tggtgattgt caagacccag    1500
cgagctgcca ttggcaattc catgttccag gaagtgagag ccattgtgag aatcagcaag    1560
aacatcgcca tcctcacacc acttctggga ctgacctggg gatttggagt agccactgtc    1620
atcgatgaca gatccctggc cttccacatt atcttctccc tgctcaatgc attccaggta    1680
agtccagatg cttctgacca agtgcaaagt gagagaattc atgaagatgt tctgtga       1737

<210> 4
<211> 578
<212> PRT
<213> homo sapiens

<400> 4
Met Gly Phe Ser Cys Arg Gln Lys Thr Trp His Lys Ile Thr Asp Thr
1               5                   10                  15
Cys Gln Thr Leu Asn Ala Leu Asn Ile Phe Glu Glu Asp Ser Arg Leu
            20                  25                  30
Val Gln Pro Phe Glu Asp Asn Ile Lys Ile Ser Val Tyr Thr Gly Lys
        35                  40                  45
Ser Glu Thr Ile Thr Asp Met Leu Leu Gln Lys Cys Pro Thr Asp Leu
    50                  55                  60
Ser Cys Val Ile Arg Asn Ile Gln Gln Ser Pro Trp Ile Pro Gly Asn
65                  70                  75                  80
Ile Ala Val Ile Val Gln Leu Leu His Asn Ile Ser Thr Ala Ile Trp
                85                  90                  95
Thr Gly Val Asp Glu Ala Lys Met Gln Ser Tyr Ser Thr Ile Ala Asn
            100                 105                 110
His Ile Leu Asn Ser Lys Ser Ile Ser Asn Trp Thr Phe Ile Pro Asp
        115                 120                 125
Arg Asn Ser Ser Tyr Ile Leu Leu His Ser Val Asn Ser Phe Ala Arg
    130                 135                 140

Arg Leu Phe Ile Asp Lys His Pro Val Asp Ile Ser Asp Val Phe Ile
145                 150                 155                 160
His Thr Met Gly Thr Thr Ile Ser Gly Asp Asn Ile Gly Lys Asn Phe
                165                 170                 175
Thr Phe Ser Met Arg Ile Asn Asp Thr Ser Asn Glu Val Thr Gly Arg
            180                 185                 190
Val Leu Ile Ser Arg Asp Glu Leu Arg Lys Val Pro Ser Pro Ser Gln
        195                 200                 205
Val Ile Ser Ile Ala Phe Pro Thr Ile Gly Ala Ile Leu Glu Ala Ser
    210                 215                 220
Leu Leu Glu Asn Val Thr Val Asn Gly Leu Val Leu Ser Ala Ile Leu
225                 230                 235                 240
Pro Lys Glu Leu Lys Arg Ile Ser Leu Ile Phe Glu Lys Ile Ser Lys
                245                 250                 255
Ser Glu Glu Arg Arg Thr Gln Cys Val Gly Trp His Ser Val Glu Asn
            260                 265                 270
Arg Trp Asp Gln Gln Ala Cys Lys Met Ile Gln Glu Asn Ser Gln Gln
        275                 280                 285
Ala Val Cys Lys Cys Arg Pro Ser Glu Leu Phe Thr Ser Phe Ser Ile
    290                 295                 300
Leu Met Ser Pro His Ile Leu Glu Ser Leu Ile Leu Thr Tyr Ile Thr
305                 310                 315                 320
Tyr Val Gly Leu Gly Ile Ser Ile Cys Ser Leu Ile Leu Cys Leu Ser
                325                 330                 335
Ile Glu Val Leu Val Trp Ser Gln Val Thr Lys Thr Glu Ile Thr Tyr
            340                 345                 350
Leu Arg His Val Cys Ile Val Asn Ile Ala Ala Thr Leu Leu Met Ala
        355                 360                 365
Asp Val Trp Phe Ile Val Ala Ser Phe Leu Ser Gly Pro Ile Thr His
    370                 375                 380
His Lys Gly Cys Val Ala Ala Thr Phe Phe Val His Phe Phe Tyr Leu
385                 390                 395                 400
Ser Val Phe Phe Trp Met Leu Ala Lys Ala Leu Leu Ile Leu Tyr Gly
                405                 410                 415
Ile Met Ile Val Phe His Thr Leu Pro Lys Ser Val Leu Val Ala Ser
            420                 425                 430
Leu Phe Ser Val Gly Tyr Gly Cys Pro Leu Ala Ile Ala Ala Ile Thr
        435                 440                 445
Val Ala Ala Thr Glu Pro Gly Lys Gly Tyr Leu Arg Pro Glu Ile Cys
    450                 455                 460
Trp Leu Asn Trp Asp Met Thr Lys Ala Leu Leu Ala Phe Val Ile Pro
465                 470                 475                 480
Ala Leu Ala Ile Val Val Val Asn Leu Ile Thr Val Thr Leu Val Ile
                485                 490                 495
Val Lys Thr Gln Arg Ala Ala Ile Gly Asn Ser Met Phe Gln Glu Val
            500                 505                 510
Arg Ala Ile Val Arg Ile Ser Lys Asn Ile Ala Ile Leu Thr Pro Leu
        515                 520                 525
Leu Gly Leu Thr Trp Gly Phe Gly Val Ala Thr Val Ile Asp Asp Arg
    530                 535                 540
Ser Leu Ala Phe His Ile Ile Phe Ser Leu Leu Asn Ala Phe Gln Val
545                 550                 555                 560
Ser Pro Asp Ala Ser Asp Gln Val Gln Ser Glu Arg Ile His Glu Asp
                565                 570                 575
Val Leu

<210> 5
<211> 2166
<212> DNA
<213> homo sapiens

<400> 5
   gaaccatcgc gcccggccca gaagatgact tcttgactcc tctctctccg tatctgacac      60
   tacttcattc tttaccagga aacaagaaag tcagctgtgt ggaagacaat gactcatata     120
   cttttgctgt actacttggt gtttcttttg cccacagagt cctgtaggac attgtatcag     180
   gctgcaagca aaagcaagga gaaggtgcct gccaggccac acggtgtatg cgatggtgtc     240
   tgtacagact acccccagtg tactcaacct tgccctccag acactcaggg aaatatgggg     300
   ttttcatgca ggcaaaagac atggcacaag atcactgaca cctgccagac tcttaatgcc 360
   ctcaacatct ttgaggagga ttcacgtttg gttcagccat ttgaagacaa tataaaaata 420
   agtgtatata ctggaaagtc tgagaccata acagatatgt tgctacaaaa gtgtcccaca 480
   gatctgtctt gtgtaattag aaacattcag cagtctccct ggataccagg aaacattgcc 540
   gtaattgtgc agctcttaca caacatatca acagcaatat ggacaggtgt tgatgaggca 600
   aagatgcaga gttacagcac catagccaac cacattctta acagcaaaag catctccaac 660
   tggactttca ttcctgacag aaacagcagc tatatcctgc tacattcagt caactccttt 720
   gcaagaaggc tattcataga taaacatcct gttgacatat cagatgtctt cattcatact 780
   atgggcacca ccatatctgg agataacatt ggaaaaaatt tcactttttc tatgagaatt 840
   aatgatacca gcaatgaagt cactgggaga gtgttgatca gcagagatga acttcggaag 900
   gtgccttccc cttctcaggt catcagcatt gcatttccaa ctattggggc tattttggaa 960
   gccagtcttt tggaaaatgt tactgtaaat gggcttgtcc tgtctgccat tttgcccaag 1020
   gaacttaaaa gaatctcact gatttttgaa aagatcagca agtcagagga gaggaggaca 1080
   cagtgtgttg gctggcactc tgtggagaac agatgggacc agcaggcctg caaaatgatt 1140
   caagaaaact cccagcaagc tgtttgcaaa tgtaggccaa gtgaattgtt tacctctttc 1200
   tcaattctta tgtcacctca catcttagag agtctgattc tgacttacat cacatatgta 1260
   ggcctgggca tttctatttg cagcctgatc ctttgcttgt ccattgaggt cctagtctgg 1320
   agccaagtga caaagacaga gatcacctat ttacgccatg tgtgcattgt taacattgca 1380
   gccactttgc tgatggcaga tgtgtggttc attgtggctt cctttcttag tggcccaata 1440
   acacaccaca agggatgtgt ggcagccaca ttttttgttc atttctttta cctttctgta 1500
   tttttctgga tgcttgccaa ggcactcctt atcctctatg gaatcatgat tgttttccat 1560
   accttgccca agtcagtcct ggtggcatct ctgttttcag tgggctatgg atgccctttg 1620
   gccattgctg ccatcactgt tgctgccact gaacctggca aaggctatct acgacctgag 1680
   atctgctggc tcaactggga catgaccaaa gccctcctgg ccttcgtgat cccagctttg 1740
   gccatcgtgg tagtaaacct gatcacagtc acactggtga ttgtcaagac ccagcgagct 1800
   gccattggca attccatgtt ccaggaagtg agagccattg tgagaatcag caagaacatc 1860
   gccatcctca caccacttct gggactgacc tggggatttg gagtagccac tgtcatcgat 1920
   gacagatccc tggccttcca cattatcttc tccctgctca atgcattcca ggtaagtcca 1980
   gatgcttctg accaagtgca aagtgagaga attcatgaag atgttctgtg attaggtgct 2040
   tgtgacatta gatttctaca taaaagccaa tggatttgga atagcatatg gagtggggaa 2100
   ataaactttt gttccagaga tacctggaaa aaaaaaaaaa aaaaaaaaaa aaaaaaaaaa 2160
   aaaaaa 2166